(19) **Europäisches Patentamt / European Patent Office / Office européen des brevets**

(11) **EP 2 667 329 A1**

(12) **EUROPEAN PATENT APPLICATION**
published in accordance with Art. 153(4) EPC

(43) Date of publication:
**27.11.2013 Bulletin 2013/48**

(51) Int Cl.:
*G06N 3/00* (2006.01)　　　　*A61B 5/117* (2006.01)
*G06F 21/20* (0000.00)　　　　*G06T 7/00* (2006.01)
*H04L 9/32* (2006.01)

(21) Application number: **12757626.2**

(22) Date of filing: **27.02.2012**

(86) International application number:
**PCT/JP2012/054754**

(87) International publication number:
**WO 2012/124458 (20.09.2012 Gazette 2012/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2011　JP 2011057523**

(71) Applicant: **NTT DOCOMO, INC.**
**Chiyoda-ku**
**Tokyo**
**100-6150 (JP)**

(72) Inventors:
 • **TSUKAMOTO, Masakatsu**
 **Tokyo 100-6150 (JP)**
 • **MORINAGA, Yasuo**
 **Tokyo 100-6150 (JP)**
 • **OTA, Manabu**
 **Tokyo 100-6150 (JP)**
 • **HIGUCHI, Takeshi**
 **Tokyo 100-6150 (JP)**

(74) Representative: **MERH-IP**
**Matias Erny Reichl Hoffmann**
**Paul-Heyse-Strasse 29**
**80336 München (DE)**

(54) **AUTHORIZED PARTY AUTHENTICATION TEMPLATE LEARNING SYSTEM AND AUTHORIZED PARTY AUTHENTICATION TEMPLATE LEARNING METHOD**

(57)　A mobile information terminal includes a sample acquisition unit configured to acquire a sample, a sample transmission unit configured to transmit the sample and a user ID, an other's sample reception unit configured to receive other's samples, a user authentication template learning unit configured to learn a user authentication template and an authentication determination threshold value by using the other's samples and samples of the authentic user, and a template storage unit configured to store the user authentication template and the authentication determination threshold value, and a server includes a sample reception unit configured to receive a sample and a user ID, a clustering unit configured to classify the sample that is received into a feature cluster, a sample storage unit configured to store the sample that is classified, in association with the user ID, a feature cluster extraction unit configured to extract all samples which belong to a feature cluster identical to a feature cluster corresponding to the user ID that is received, and an other's sample transmission unit configured to transmit the samples that are extracted, as other's samples.

FIG. 6

**Description**

[TECHNICAL FIELD]

The present invention relates to a user authentication template learning system and a user authentication template learning method for learning a user authentication template which is used for biometric authentication of a mobile information terminal.

[BACKGROUND ART]

In recent years, various services related to money such as electronic money have become widespread along with improvement of functionality of a mobile information terminal. Further, more information related to personal privacy such as address books, mails, pictures, and website browse history has become to be recorded in a mobile information terminal along with the improvement of functionality of the mobile information terminal. In related art, security for information which is managed in a mobile information terminal has been ensured by user authentication using biometric information in a use of a mobile information terminal. One example of the known user authentication section, which uses biometric information, for mobile information terminals is a fingerprint authentication section included in a mobile information terminal disclosed in Patent Literature 1. Patent Literature 1 discloses a fingerprint-authentication-capable mobile information terminal that includes a sending and receiving section for communicating with another mobile information terminal, a fingerprint reading section, a fingerprint authentication section for authenticating a read fingerprint, and a section for omitting fingerprint authentication in a predetermined period of time after successful fingerprint authentication, in which communication with another mobile information terminal is activated only if a user of the mobile information terminal is validated as the authentic owner of the mobile information terminal through successful fingerprint authentication and the sending and receiving section is deactivated after the expiration of the period in which the authentication is omitted. Since the mobile information terminal in Patent Literature 1 is configured as described above, the functionality of the mobile information terminal is disabled before the fingerprint authentication of the authentic owner to prevent a criminal use of the mobile information terminal by a malicious other's while at the same time the conventional convenience of the mobile information terminal is maintained by setting an appropriate authentication omission period after fingerprint authentication is performed.

[PRIOR ART LITERATURE]

[PATENT LITERATURE]

Patent literature 1: Japanese Patent Application Laid Open No. 2010-128600

[SUMMARY OF THE INVENTION]

[PROBLEMS TO BE SOLVED BY THE INVENTION]

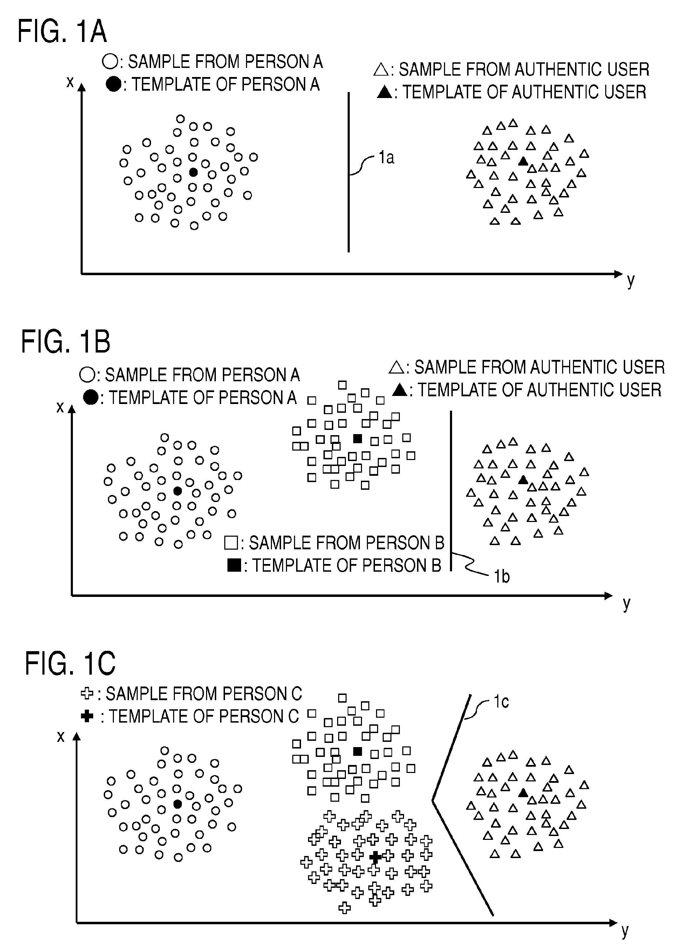

A user authentication method using biometric information (fingerprint, finger vein, iris, or the like) such as that in Patent Literature 1 is executed by comparing a sample which is acquired by a sensor in authentication with information which is called a template which is generated from biometric information and is preliminarily registered. Many techniques of pattern recognition are used in this comparison. In pattern recognition, a degree of similarity between a template and a sample is obtained by using an inter-vector distance. Examples of a distance which is often used in biometric authentication include Mahalanobis's generalized distance and Hamming distance. When these distances exceed a predetermined threshold value, it is determined that a sample is someone's. On the other hand, when these distances do not exceed the threshold value, it is determined that the sample is user's. This is specifically described with reference to Figs. 1A to 1C.
Figs. 1A to 1C illustrate a template, a sample, and a threshold value in pattern recognition of a case of a two-dimensional feature pattern. In Fig. 1A, samples from another person A, a template of the person A, samples from an authentic user, and a template of the authentic user are respectively denoted by white circles, a black circle, white triangles, and a black triangle, on a common x-y coordinate. In this case, a threshold value 1a is set so that an authentic user is not rejected in mistake for the person A even in a case of a sample which is farthest from the template of the authentic user among the samples of the person A and the authentic user (a rate for mistaking an authentic user as another person in user authentication is referred to as a false rejection rate) and also, another person is not rejected in mistake for the authentic user even in a case of a sample which is closest to the template of the authentic user (a rate for mistaking another person as an authentic user in user authentication is referred to as a false acceptance rate). Here, it is assumed that still another person B depicted in Fig. 1B appears on the premise of the relation of Fig. 1A. Samples from the person B and a template of the person B are respectively denoted by white quadrangles and a black quadrangle. When another person having a template which is closer to the authentic user appears as the person B, it is necessary to update the threshold value 1a in Fig. 1A with a threshold value 1b of Fig. 1B so as to prevent increase of the false acceptance rate. Further, it is assumed that yet another person C depicted in Fig. 1C appears on the premise of the relation of Fig. 1B. Samples from the person C and a template of the person C are respectively denoted by white crosses and a black cross. In this case, by setting threshold values for respective templates of the person B and the person C with respect to the template of the authentic user, a threshold value can be optimally set as a threshold value 1c (multi-template method). Further, not limited to the method of Figs. 1A to 1C, when the template of the authentic user is learned, for example, a position which is separated from the template of the authentic user by a predetermined distance may be set as a threshold value so as to omit collection of samples of other people.

In order to set an above-described threshold value, it is necessary to record samples of a large indefinite number of persons which are preliminarily collected in manufacturing as other's samples or omit collection of other's samples to preliminarily set a distance between a template and a threshold value to an appropriate value, for example. However, these pieces of information are incorporated in a mobile information terminal in a manufacturing stage, making update difficult. Even if it is possible to update these pieces of information, it is necessary to allow other people to use user's own mobile information terminal so as to collect new other's samples required for update, being unfavorable from a viewpoint of security. Further, in a case where the above-mentioned distance between a template and a threshold value is preliminarily set, as well, it is necessary to collect new other's samples and check transition of a false acceptance rate and a false rejection rate at the set value after update so as to update the set value to an optimum value. Consequently, a problem on security arises in collection of other's samples in a similar manner to the above description. The object of the present invention is to provide a user authentication template learning system in which other's samples required for update of a threshold value or learning (re-learning) of a template are recorded in a server and the samples can be appropriately supplied from the server to a mobile information terminal.

[MEANS TO SOLVE THE PROBLEMS]

A user authentication template learning system according to the present invention includes two or more mobile information terminals and a server. Each of the mobile information terminals comprises a sample acquisition unit configured to acquire a sample which is used for biometric authentication, a sample transmission unit configured to transmit the acquired sample along with a user ID for specifying the mobile information terminals to the server, an other's sample reception unit configured to receive other's samples from the server, a user authentication template learning unit configured to perform learning of a user authentication template and an authentication determination threshold value by using the other's samples and samples of the authentic user, and a template storage unit configured to store the user authentication template that is learned.

The server comprises a sample reception unit configured to receive a sample and a user ID from each of the mobile information terminals, a clustering unit configured to classify each sample that is received into any one of two or more predetermined feature clusters, a sample storage unit configured to store the sample that is classified in association with the user ID that is received, a feature cluster extraction unit configured to extract all samples belonging to a feature cluster identical to the feature cluster corresponding to the user ID that is received, other than the sample corresponding to the user ID that is received, from the sample storage unit, and an other's sample transmission unit configured to transmit the samples that are extracted, as other's samples, to the mobile information terminal that has transmitted the user ID.

[EFFECTS OF THE INVENTION]

According to the user authentication template learning system of the present invention, other's samples required for update of a threshold value or learning (re-learning) of a template can be stored in a server and the other's samples can be properly supplied from the server to the mobile information terminals.

[BRIEF DESCRIPTION OF THE DRAWINGS]

Fig. 1A illustrates an example of a template, a sample, and a threshold value in pattern recognition of a case of a two-dimensional feature pattern;

Fig. 1B illustrates another example of a template, a sample, and a threshold value in pattern recognition of a case of a two-dimensional feature pattern;

Fig. 1C illustrates yet another example of a template, a sample, and a threshold value in pattern recognition of a case of a two-dimensional feature pattern;

Fig. 2 illustrates a state in which a portable terminal according to all embodiments is gripped;

Fig. 3 illustrates a case where a portable terminal according to all the embodiments includes a pressure sensor array;

Fig. 4 illustrates a case in which the portable terminal according to all the embodiments includes a fingerprint authentication sensor;

Fig. 5 illustrates samples which are stored by a server according to all the embodiments;

Fig. 6 is a block diagram illustrating the configuration of a user authentication template learning system according to a first embodiment;

Fig. 7 is a flowchart illustrating an operation in sample registration of the user authentication template learning system according to the first embodiment;

Fig. 8 is a flowchart illustrating an operation in other's sample request of the user authentication template learning system according to the first embodiment;

Fig. 9 is a block diagram illustrating the configuration of a user authentication template learning system according to a second embodiment;

Fig. 10 is a flowchart illustrating an operation in sample registration of the user authentication template learning system according to the second embodiment;

Fig. 11 is a flowchart illustrating an operation in other's sample request of the user authentication template learning system according to the second embodiment;

Fig. 12 is a block diagram illustrating the configuration of a user authentication template learning system according to a third embodiment;

Fig. 13 is a flowchart illustrating an operation in sample registration of the user authentication template learning system according to the third embodiment;

Fig. 14 is a flowchart illustrating an operation in other's sample request of the user authentication template learning system according to the third embodiment;

Fig. 15 is a block diagram illustrating the configuration of a user authentication template learning system according to a fourth embodiment;

Fig. 16 is a flowchart illustrating an operation in sample registration of the user authentication template learning system according to the fourth embodiment; and

Fig. 17 is a flowchart illustrating an operation in other's sample request of the user authentication template learning system according to the fourth embodiment.

## [DETAILED DESCRIPTION OF THE EMBODIMENTS]

Embodiments of the present invention will be described in detail below. Constituent elements having the same functions as each other are given the same reference characters and duplicate description will be omitted.

<About mobile information terminal>

Examples of a device embodying a mobile information terminal of the present invention include a portable terminal, a PDA, a portable game device, an electronic organizer, an electronic book reader, and the like. However, in addition to or in place of these devices, any other devices that meet requirements (1) through (4) given below can be used as mobile information terminals of the present invention: a device that (1) is capable of acquiring a sample used for biometric authentication while being used, (2) is connected via a server and a network and is capable of transmitting/receiving data, (3) has a risk of leakage of personal information and value information due to loss or theft, and (4) is used while being carried and of which a position changes at each time. In the following descriptions of embodiments, a portable terminal will be taken as a specific example and explained in detail.

<Sample used for biometric authentication -gripping feature->

First, samples which are acquired by portable terminals 600, 600', and 600" according to all embodiments of the present invention and are used for biometric authentication (also referred to as biometric authentication samples or merely as samples, below) will be described.

A gripping feature sample, for example, can be used as a sample used for biometric authentication. Since human beings are innately different in (1) the lengths of their fingers and (2) the strength of their gripping force and, as an acquired nature, in (3) the habit of gripping a portable terminal, gripping-features are extremely suitable as biometric information used for user authentication. More specifically, gripping-feature authentication has almost the same level of precision as general face authentication in terms of the false rejection rate and the false acceptance rate. Examples of a gripping-feature sample can include gripping-pressure distribution, gripping-shape distribution, and gripping-heat distribution. As an example method of acquiring these gripping-feature samples, the gripping-pressure distribution can be acquired by distributing pressure sensor elements in an array on the portable terminals 600, 600', and 600". In the same manner, the gripping-shape distribution can be obtained by distributing CCD (CMOS) sensor elements in an array. In the same manner, the gripping-heat distribution can be obtained by distributing infrared sensor elements in an array. In a case of a portable terminal which has operating keys at the rear surface thereof (touch sensitive panel), gripping-features can be acquired even from pressing states (whether the operating keys or the touch sensitive panel is pressed) of the operating keys (touch sensitive panel) when the terminal is gripped. In the following descriptions of the embodiments, gripping-pressure distribution will be taken as a specific example of a gripping-feature sample.

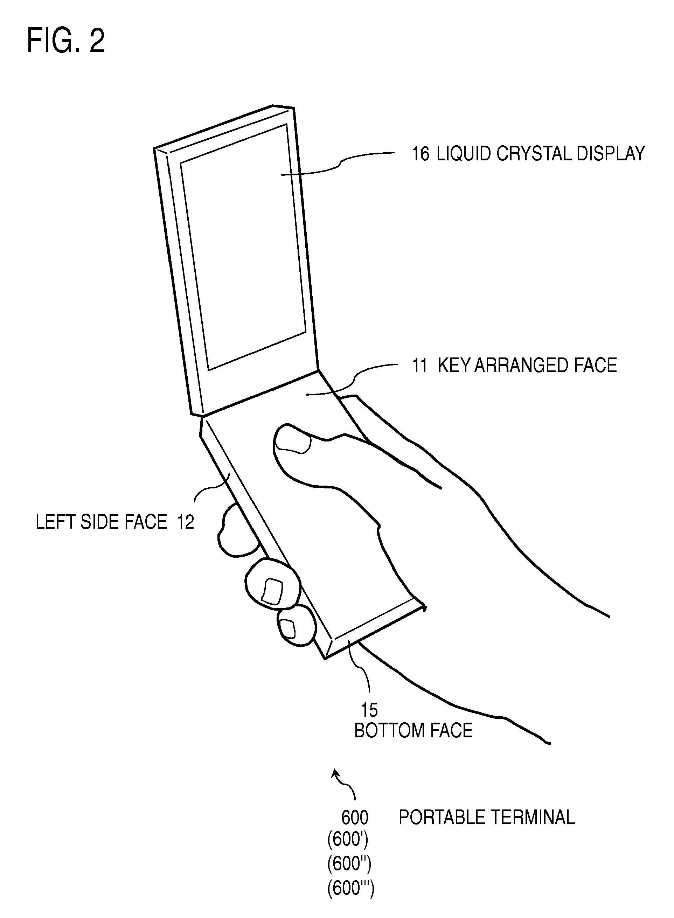

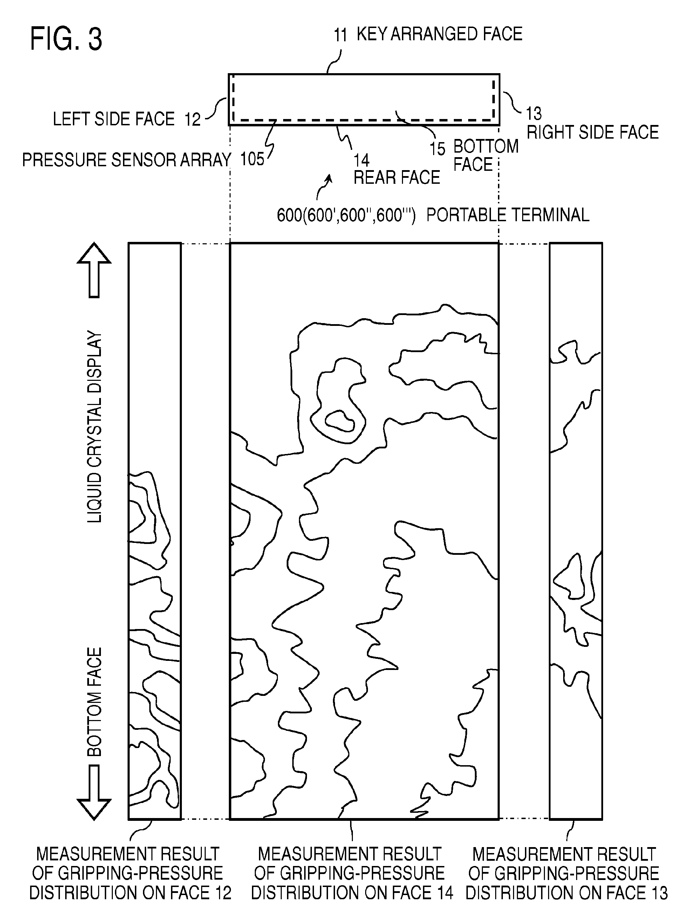

Acquisition of gripping-pressure distribution by using a pressure sensor array will be described in detail with reference to Fig. 2 and Fig. 3. Fig. 2 illustrates a state in which the portable terminal 600, 600', or 600" according to any one of all the embodiments is gripped. Fig. 3 illustrates a case where the portable terminals 600, 600', and 600" according to all the embodiments include a pressure sensor array 105. It is assumed here that the portable terminals 600, 600', and 600" are general folding-type portable terminals. Two long-plate-shaped bodies are foldably coupled with a coupling shaft at short sides of the respective bodies. One of the bodies has operating keys arranged. A surface on which the operating keys are arranged is called a key arranged face 11, side faces at the left and right of the key arranged face 11 are called a left side face 12 and a right side face 13, a back surface of the key arranged face 11 is called a rear face 14, and a side face of the key arranged face 11 at the bottom (face opposite to the face where the coupling shaft is placed) is called a bottom face 15. Further, a liquid crystal display 16 is provided in the other body on a surface facing the key arranged face 11 when the terminal is folded.

The portable terminals 600, 600', and 600" are configured as described above, but the foregoing description explains merely an example for describing in detail the gripping-pressure distribution outputted from a pressure sensor array which is to be described later. Therefore, the portable terminals 600, 600', and 600" are not necessarily folding-type terminals, such as that illustrated in Fig. 2, and can have any shapes, such as a straight type and a sliding type. Here, it is assumed that a user of the portable terminals 600, 600', and 600" grips the portable terminals 600, 600', and 600" as depicted in Fig. 2. A pressure sensor array 105 (indicated by a dotted line in Fig. 3) is arranged so as to be able to detect external gripping pressure, on the surface of the body where the key arranged face 11 of the portable terminals 600, 600', and 600" is arranged. The pressure sensor array 105 is capable of detecting the gripping-pressure distribution on the left side face 12, the right side face 13, and the rear face 14 of the portable terminals 600, 600', and 600". When signals sent from respective pressure sensors of the pressure sensor array 105 are analyzed, gripping-pressure distribution such as that depicted in Fig. 3 can be drawn. It is understood from the gripping-pressure distribution depicted in Fig. 3 that characteristics of the fingers and the gripping force of the user are exhibited clearly on the left side face 12, the right side face 13, and the rear face 14. The gripping-pressure distribution acquired in this way can be used as samples used for biometric authentication in the present invention.

<User authentication and authentication threshold value>

Examples of a distance serving as a determination criterion of user authentication, described earlier, will be explained below. For example, a pressure value which is acquired by the i-th sensor element in the j-th measurement performed for learning is denoted as $x_{i,j}$. Here, i=1, 2, ..., n, j=1, 2, ..., m, n denotes the maximum number of sensor elements and is an integer equal to 2 or greater, and m denotes the maximum number of times of acquisition of gripping-feature samples for learning and is an integer equal to 2 or greater. An average of pressure values, variance, and vectors of the average and the variance are defined as follows:

$$\overline{x}_i = \frac{1}{m}\left(\sum_{j=1}^{m} x_{i,j}\right)$$

$$s_i^2 = \frac{1}{m}\sum_{j=1}^{m}(\overline{x}_i - x_{i,j})^2$$

$$X = (\overline{x}_1, \overline{x}_2, \cdots, \overline{x}_n); \quad S^2 = (s_1^2, s_2^2, \cdots, s_n^2)$$

An average vector of the gripping-feature samples is used as a user authentication template. The user authentication template will be indicated with a subscript "le". The Mahalanobis's generalized distance $f_1$ is expressed by the following formula.

$$f_1 = \left[ \sum_{i=1}^{n} \left( \frac{x_i - {}_{le}\overline{x}_i}{s_i} \right)^2 \right]^{1/2}$$

As another example of a distance, the Euclidean distance $f_2$ is defined by the following formula.

$$f_2 = \left[ \sum_{i=1}^{n} (x_i - {}_{le}\overline{x}_i)^2 \right]^{1/2}$$

As still another example of a distance, the Manhattan distance $f_3$ is defined by the following formula.

$$f_3 = \sum_{i=1}^{n} | x_i - {}_{le}\overline{x}_i |$$

Any one of these three distances can be used to perform determination with the following determination formula in common. Data of the user-to-be-authenticated, acquired for determination, will be indicated with a subscript "self", and data of other people will be indicated with a subscript "oth". When the threshold value used to determine other people is defined as $x_{thre}$, the following formula can be used to determine other people.

$$x_{thre} < {}_{oth}f$$

It is assumed here that gripping-feature sample data of other people is available in some method, such as embedding the data in the portable terminal in advance, allowing the user to access the data on the Internet, or allowing the user to acquire the data by asking other people to grip the portable terminal. From the data of other people and the user authentication template, the distance ${}_{oth}f$ is calculated. The threshold value $x_{thre}$ is determined to satisfy the following condition after the distance ${}_{self}f$ is calculated from a gripping-feature sample of the authentic user, not used for template learning, and the learned template.

$$_{self}f < x_{thre} < {}_{oth}f$$

The user authentication template is obtained from the average of gripping-feature samples in the foregoing description. However, other methods can be used. For example, pressure distribution acquired from the sensor elements at n points is divided into appropriate areas (10 areas, for example, where n is larger than 10); a sum (or an average) of gripping pressure in each of the areas is calculated to generate vector data composed of, as vector elements, the sums (or the averages) of gripping pressures in the respective areas; and such vector data is generated for each of m gripping-feature samples, and the average thereof is used as a template. Alternatively, positions of sensor elements having the top 20 pressure values among the n sensor elements are recorded; vector data thereof is generated; and such vector data is generated for each of m gripping-feature samples, and the average thereof is used as the template.

<Sample used for biometric authentication -fingerprint->

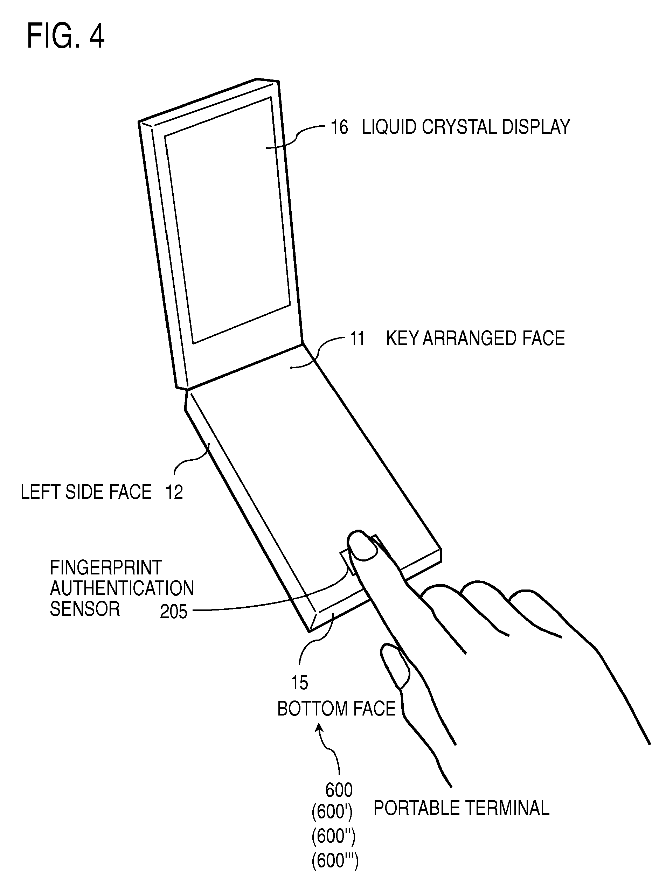

As a sample used for biometric authentication, a fingerprint may be used other than the above-mentioned gripping-feature sample, for example. An example of a portable terminal provided with a fingerprint authentication sensor is illustrated in Fig. 4. Fig. 4 illustrates a case in which the portable terminal 600, 600', or 600" according to any one of all the embodiments includes a fingerprint authentication sensor 205. As illustrated in Fig. 4, a terminal having the configuration in which the fingerprint authentication sensor 205 is provided on an edge part of a key arranged face 11 of a portable  terminal of related art is known. As another example, there is a portable terminal which is provided with a fingerprint authentication sensor on a rear face 14 side of the portable terminal. In pattern recognition based on a fingerprint, an end point/a branch point (called minutia) of a fingerprint ridge which is a local feature of a fingerprint image is extracted as a feature point, for example, or directions of a fingerprint ridge is classified and extracted into eight directions or 16 directions for every partial region of a fingerprint, so as to be used for template generation.

<Sample used for biometric authentication -others->

As samples used for biometric authentication, many biometric authentication techniques other than that using the above-mentioned gripping-feature sample and fingerprint are known. For example, a finger vein pattern may be used as a sample used for biometric authentication of the present invention. The finger vein authentication is realized such that pattern recognition is performed by using an image of a finger vein pattern which is obtained by transmitting near infrared to a finger. Further, iris authentication may be used as a sample used for biometric authentication of the present invention, for example. An iris is a thin membrane surrounding a pupil and has a function corresponding to a diaphragm of a camera. Iris authentication is an authentication method using a histogram of a gray value of an iris. Other than this, voiceprint authentication, face authentication, and the like may be used as a sample for biometric authentication of the present invention. An authentication method which has high reliability and can be incorporated in a small-sized device such as a portable terminal is favorably used as a biometric authentication method used in the present invention. However, an  advantageous effect of the present invention can be realized by using any biometric authentication.

<Registration of samples of other people in server>

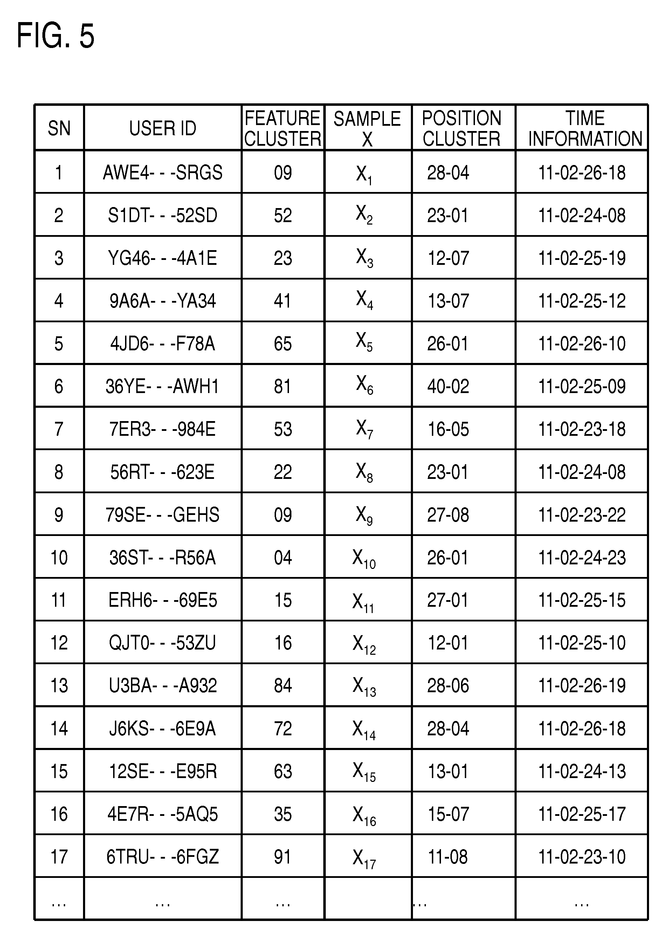

In the user authentication template learning system of the present invention, samples for biometric authentication are collected from many users of portable terminals via a network, and the collected samples are collectively managed in a sample storage unit which is included in a server. Here, samples which are collectively managed by a server do not have to be samples themselves which are collected from respective individuals. A distribution model of samples may be estimated on the basis of an average and variance of samples while assuming samples are based on normal distribution, for example, and samples which are randomly generated in a pseudo manner in accordance with the distribution model may be collectively managed. Further, data which is obtained by arbitrarily processing and modifying samples which are collected from respective individuals may be collectively managed, for example. In this sample storage unit, samples are registered in a manner to be classified based on a later-described cluster and time information. This will be described in detail below with reference to Fig. 5.

<User ID>

Fig. 5 illustrates samples which are stored by servers 700, 700', and 700" according to all the embodiments. Samples which are collected from a user of a portable terminal are provided with serial numbers and managed by a list as illustrated in Fig. 5. In the list, user identifiers (referred to below as user IDs) of portable terminals which transmit the samples to the server are  recorded. A user ID may be an identification number of a user who has registered use of the portable terminal on a network service provider or may be a manufacturing number of the portable terminal used. Fig. 5 illustrates an example which uses a terminal manufacturing number (a part of the number is replaced with "-" symbols to be hidden in Fig. 5) composed of 11-digit alphanumeric numbers, as a user ID. However, a user ID is not limited to this example and may include any kind of identification information by which a portable terminal of a user can be uniquely specified, such as a telephone number of a subscriber and a number related to contract information.

<Feature cluster>

Samples which are collected in a server are classified depending on a feature cluster. A feature cluster represents a number which is defined for every feature of a sample so as to classify biometric authentication samples, of which features are similar to each other, into the same cluster. For example, when a gripping-feature sample is taken as an example, there are several groups in a way to grip. The way to grip can be classified into many groups such as a group of a manner of gripping a portable terminal in which an index finger, a middle finger, a ring finger, and a pinky finger are brought into contact with a rear face of the portable terminal in an aligned fashion, a group of a manner of gripping a portable terminal while hanging only an index finger on a side face, and a group of a manner of gripping a portable terminal while hanging a pinky finger on a corner of a bottom face of the portable terminal, for example, and a distinctive feature appears in a shape of gripping-pressure distribution for every group of these ways to grip. As an example of the way of dividing into feature clusters, a measurement surface of gripping pressure is divided into a plurality of predetermined areas and feature cluster classification is performed on the basis of a combination of an area number for specifying an area in which the strongest gripping force is detected and the number of areas in which gripping force which is equal to or larger than a prescribed value is detected. For example, when the number of division areas is 9, the maximum number of areas in which the maximum gripping force can be detected is 9 and the maximum number of areas in which gripping power which is equal to or larger than a prescribed value is detected is also 9, allowing to divide into $9 \times 9 = 81$ feature clusters.

Further, in the fingerprint authentication, there are main categories in fingerprint ridges such as a group of a type in which a fingerprint ridge is composed of a spiral line (whorl pattern), a group of a type in which a fingerprint ridge is in a shape of a hoof (hoop pattern), a group of a type in which a fingerprint ridge is composed only of a bow-shaped line (arch pattern). In addition to these main categories, the arch pattern is classified into the first category to the fourth category depending on features of details, for example. Thus, features of samples which are used for biometric authentication can be classified by whether or not to have a predetermined feature. Numbers are preliminarily given to categories (these numbers are referred to as feature clusters) as depicted in Fig. 5 and thus, samples which are collected can be classified into feature clusters depending on a feature possessed by the samples.

<Position cluster>

Samples which are collected in a server can be classified on the basis of position clusters, as well. A position cluster represents a number which is used for defining position information of a portable terminal at the time of sample transmission by the portable terminal, for every predetermined area so as to classify biometric authentication samples, of which geographical positions are similar to each other, into the same cluster. A position cluster can be set minutely in accordance with latitude information and longitude information. However, the number of samples which are included in the same position cluster decreases along with increase of the number of position clusters, so that it is not favorable to extremely segmentalize position clusters. For example, as a position cluster, samples can be classified in the level of a city or a ward depending on population density of the corresponding city, as depicted in Fig. 5. A sample of the serial number 1 is classified into the position cluster 28-04 (Chuo-ku, Kobe-city), for example. "28" presented before a hyphen is obtained by numbering "Hyogo-prefecture" among prefectures and "04" presented after the hyphen is obtained by numbering "Chuo-ku, Kobe-city" which is a section defined on the basis of demographic movement in Hyogo-prefecture. Further, the classification method is not limited to that in Fig. 5, and position clusters which are numbered depending on prefectural division or position clusters which are numbered depending on local division such as Hokkaido-area, Tohoku-area, and Kanto-area may be used.

<Time information>

Samples of other people which are collected in a server may be classified on the basis of time information, as well. Time information represents a record of time at which a portable terminal transmits a sample to a server. In the present invention, every time a portable terminal acquires a biometric authentication sample from a user, the portable terminal records the time as time information and transmits the time information with the biometric authentication sample to the server. Time information can be recorded on an hour basis, for example, as depicted in Fig. 5. Other than this, time information can be recorded on a minute basis or a day basis. In the present invention, time information is used in sets with a position cluster which has been described above. Time information is acquired to extract samples of other people who may be on positions near the user on the same time and to locally optimize a user authentication template by using the samples of other people. Accordingly, it is favorable that time information is deep enough to enable proper estimation of a possibility of whether or not people other than a user have been on positions near the user on the same time.

Here, only a user ID and a feature cluster are used in a first embodiment of the present invention described below. Only a user ID and a position cluster are used in a second embodiment. A user ID, a position cluster, and time information are used in a third embodiment. A user ID, a feature cluster, a position cluster, and time information are used in a fourth embodiment.

[First embodiment]

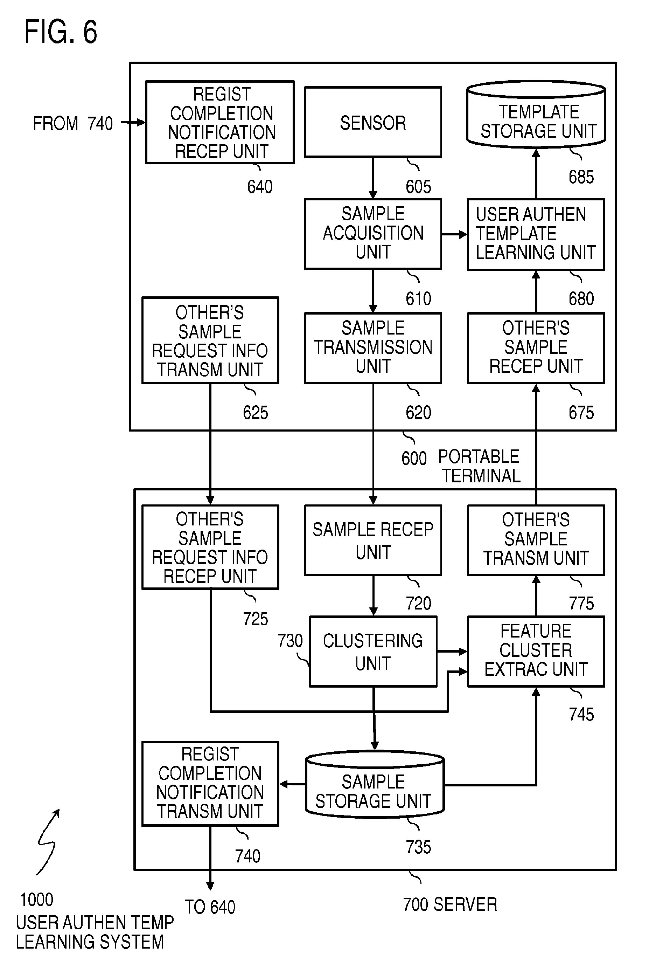

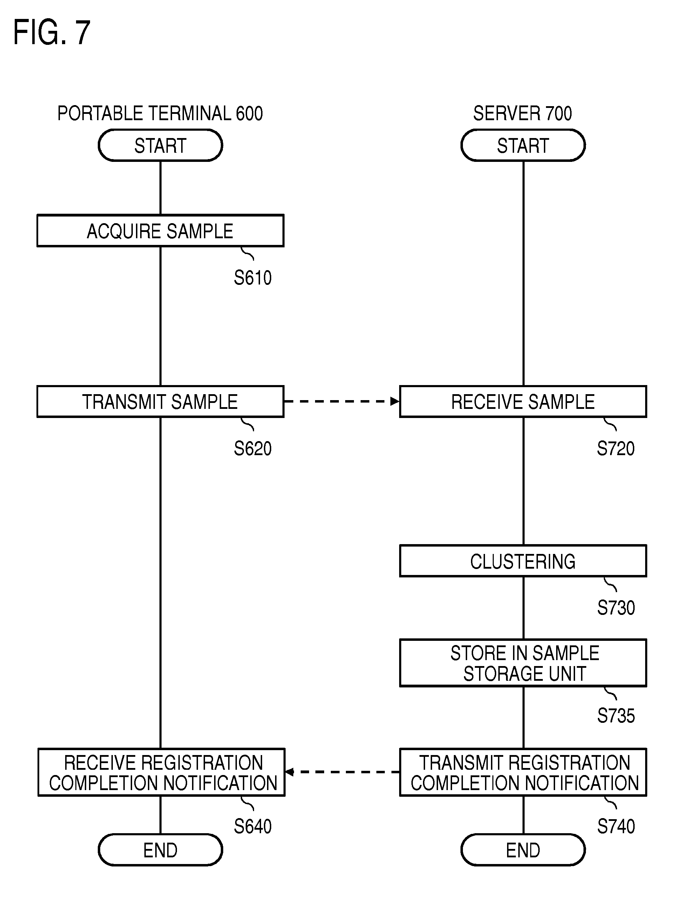

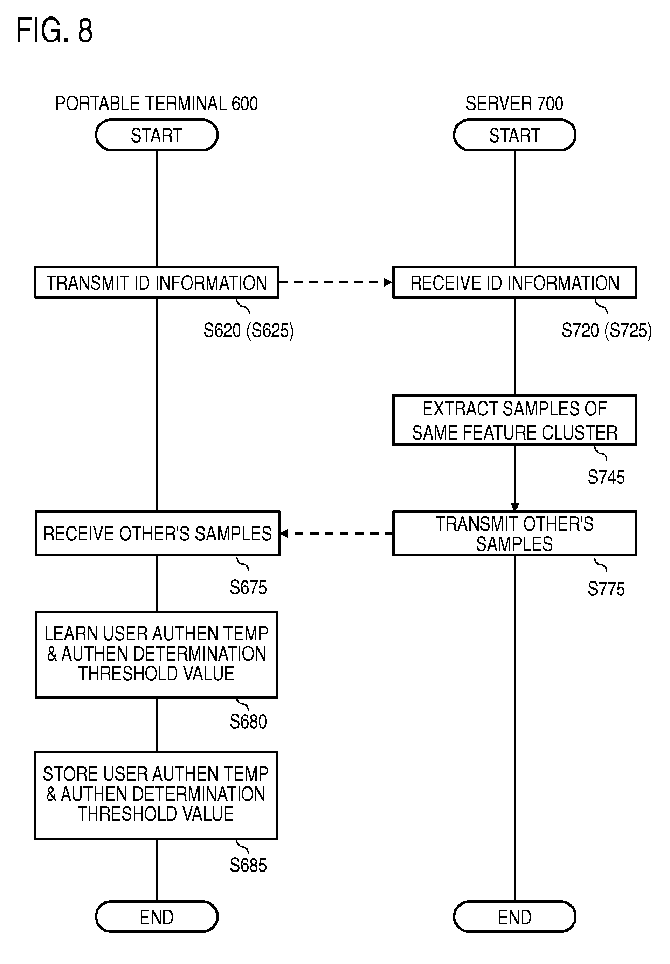

A user authentication template learning system according to the first embodiment is will be described in detail with reference to Figs. 6, 7, and 8. Fig. 6 is a block diagram illustrating the configuration of a user authentication template learning system 1000 according to the embodiment. Fig. 7 is a flowchart illustrating an operation in sample registration of the user authentication template learning system 1000 according to the embodiment. Fig. 8 is a flowchart illustrating an operation in other's sample request of the user authentication template learning system 1000 according to the embodiment. The user authentication template learning system 1000 according to the embodiment includes the portable terminal 600 and the server 700. The portable terminal 600 includes a sensor 605, a sample acquisition unit 610, a sample transmission unit 620, an other's sample request information transmission unit 625, a registration completion notification reception unit 640, an other's sample reception unit 675, a user authentication template learning unit 680, and a template storage unit 685. As the sensor 605 used in the embodiment, the pressure sensor array 105 described with reference to Fig. 3 or the fingerprint authentication sensor 205 described with reference to Fig. 4 may be used. Any device may be used as the sensor 605 as long as the device is capable of acquiring a sample which is used for biometric authentication and is small enough to be incorporated in a portable terminal. On the other hand, the server 700 includes a sample reception unit 720, an other's sample request information reception unit 725, a clustering unit 730, a sample storage unit 735, a registration completion notification transmission unit 740, a feature cluster extraction unit 745, and an other's sample transmission unit 775.

An operation of the user authentication template learning system 1000 when the portable terminal 600 transmits a biometric authentication sample thereof to the server 700 so as to obtain registration of the biometric authentication sample will be first described. The sample acquisition unit 610 acquires a sample which is to be used for biometric authentication from the sensor 605 (S610). The sample transmission unit 620 transmits the acquired sample with a user ID for specifying a portable terminal to the server 700 (S620). On the other hand, the sample reception unit 720 of the server 700 receives the sample and the user ID from the portable terminal 600 (S720). The clustering unit 730 classifies the received sample to any of two or more predetermined feature clusters (S730). The sample storage unit 735 stores the classified sample and the feature cluster of the sample while associating the sample and the feature cluster with the received user ID (S735). Accordingly, a position cluster and time information illustrated in Fig. 5 are not used in this embodiment. When a sample is stored in the sample storage unit 735, the registration completion notification transmission unit 740 transmits registration completion notification to the portable terminal 600 (S740). The registration completion notification reception unit 640 of the portable terminal 600 receives the registration completion notification from the server 700 (S640). Thus, when the portable terminal 600 obtains registration of a biometric authentication sample thereof, each biometric authentication sample is classified and stored by using a feature cluster which is defined in accordance with the biometric authentication sample in the server 700. Therefore, biometric authentication sample of which features are similar to each other are stored in the same feature cluster (refer to a section of <Feature cluster> for details).

Subsequently, an operation of the user authentication template learning system 1000 when the portable terminal 600 requests other's samples from the server 700 will be described. A method by which the portable terminal 600 acquires other's samples from the server 700 can be set as following, for example.

<(A) Automatic acquisition in registration>

The simplest method for acquiring other's samples is a method in which a user of the portable terminal 600 registers a biometric authentication sample of his/her own on the server 700 and the other's samples are transmitted from the server 700 to the portable terminal 600 at time when the server 700 transmits registration completion notification to the portable terminal 600 (S740). In this case, reception of a user ID which is simultaneously transmitted by the portable terminal 600 in the transmission of the biometric authentication sample for registration causes the server 700 to transmit all samples, which belong to a cluster to which the biometric authentication sample for registration belongs, other than the sample of the authentic user as other's samples.

<(B) Manual acquisition by user>

A user of the portable terminal 600 can acquire other's samples manually, as well. In this case, when the user of the portable terminal 600 performs a predetermined manual acquisition request operation on the portable terminal 600, the portable terminal 600 transmits the user ID to the server 700. Reception of the user ID which is transmitted by the portable terminal 600 causes the server 700 to acquire a feature cluster of biometric authentication samples which are registered by a user ID same as this received user ID from the sample storage unit 735 and transmit all samples, which belong to the cluster same as this feature cluster, other than the sample of the user, as other's samples.

<(C) Automatic acquisition by portable terminal 600 which satisfies certain conditions>

It can be set that the portable terminal 600 which satisfies certain conditions automatically acquires other's samples. Certain conditions can be defined as the following, for example. (a) A distance between a place on which the last user authentication template is formed and a current position is equal to or longer than a specific distance (for example, the portable terminal has been moved from Kanto area to Kansai area). (b) A time interval equal to or longer than a certain interval of time has elapsed from time and date on which the last user authentication template has been generated (for example, one month or longer has elapsed from generation of the last user authentication template). (c) A false acceptance rate (FAR) and a false rejection rate (FRR) have deteriorated (for example, both of the FAR and the FRR have deteriorated from 2% to 5%). When at least one condition among these conditions is satisfied, the portable terminal 600 transmits the user ID to the server 700. Reception of the user ID which is transmitted by the portable terminal 600 causes the server 700 to acquire a feature cluster of biometric authentication samples which are registered by a user ID same as this received user ID from the sample storage unit 735 and transmit all samples, which belong to the cluster same as this feature cluster, other than the sample of the user, as other's samples.

Thus, there are various methods as a method in which the portable terminal 600 acquires other's samples from the server 700, and the method is not limited to the above-mentioned methods (A) to (C). For the sake of simplicity, a trigger by which the portable terminal 600 requests other's samples from the server 700 is "reception of a user ID by the server 700" in any method.

As described above, in a case of the other's sample acquisition method (A), in response to the reception of a user ID with samples from the sample transmission unit 620 (S720), an other's sample transmission operation is started. In the case of the other's sample acquisition method (B), the other's sample request information transmission unit 625 of the portable terminal 600 transmits other's sample request information including the user ID to the server 700 after reception of registration completion notification (S625), and the other's sample request information reception unit 725 of the server 700 receives the other's sample request information including the user ID from the portable terminal 600 (S725). In response to this reception operation, an other's sample transmission operation is performed. The feature cluster extraction unit 745 extracts all the samples, which belong to a feature cluster identical to the feature cluster to which the samples received along with the user ID by the sample reception unit 720 (or a feature cluster which is registered by a user ID same as the user ID included in the other's sample request information which is received by the other's sample request information reception unit 725, in the case of the method (B)) belongs, other than the sample of the user from the sample storage unit 735 in the case of the method (A) (S745). The other's sample transmission unit 775 transmits the extracted samples as other's samples to the portable terminal 600 which has transmitted the user ID (S775). On the other hand, the other's sample reception unit 675 of the portable terminal 600 receives the other's samples from the server 700 (S675). The user authentication template learning unit 680 performs learning (re-learning) of the user authentication template and an authentication determination threshold value by using the other's samples and the sample of the user (S680). The template storage unit 685 stores the learned user authentication template and authentication determination threshold value (S685). In this learning, a user authentication template is determined by using samples of the user as described above and a threshold value which is used for performing user authentication is further determined through learning by using other's samples and the user samples. Alternatively, in a case where a distance between a sample and an authentication template of a user is compared with a distance between the sample and an authentication template of other people so as to determine the user or other people on the basis of the closer template, as a method of user authentication, an other's authentication template is generated through learning on the basis of the other's samples which are received.

Thus, in the user authentication template learning system 1000 of the embodiment, real other's samples of which a feature is similar to the sample of the user which has been registered on a server can be acquired so as to learn (re-learn) a user authentication template which has been learned and an authentication determination threshold value, enabling to improve accuracy of biometric authentication.

[Second embodiment]

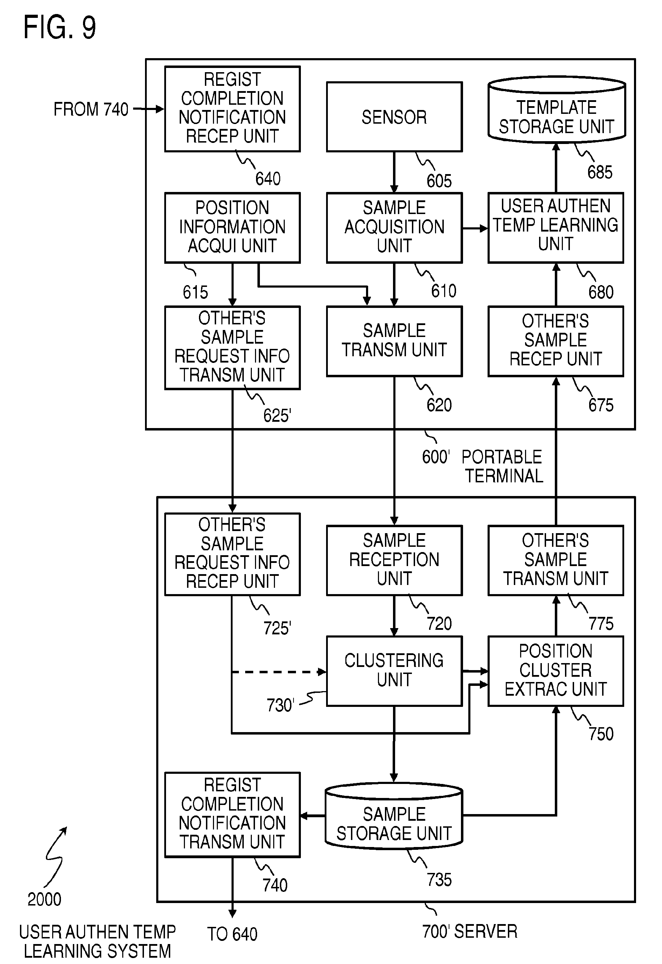

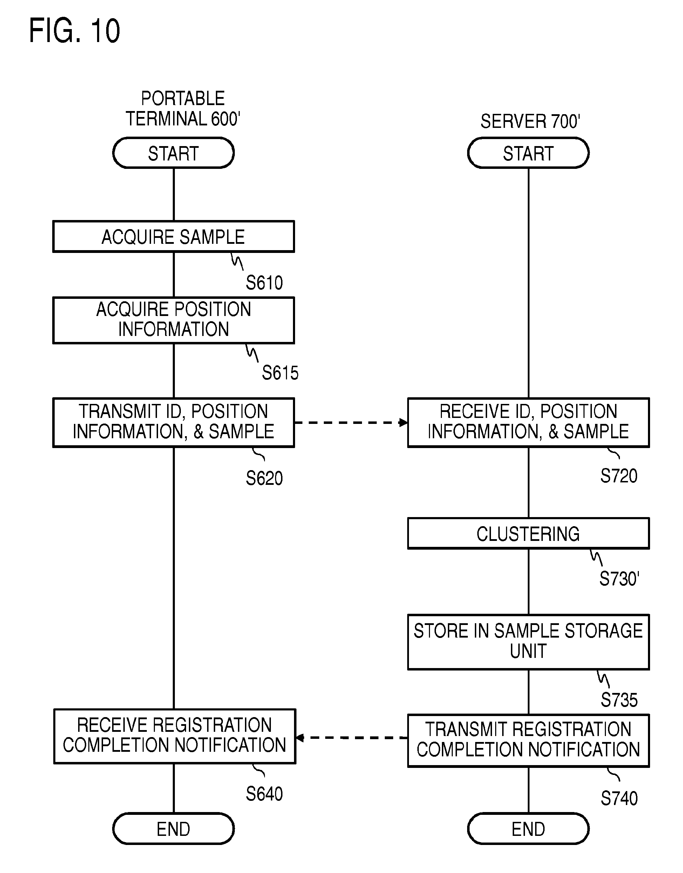

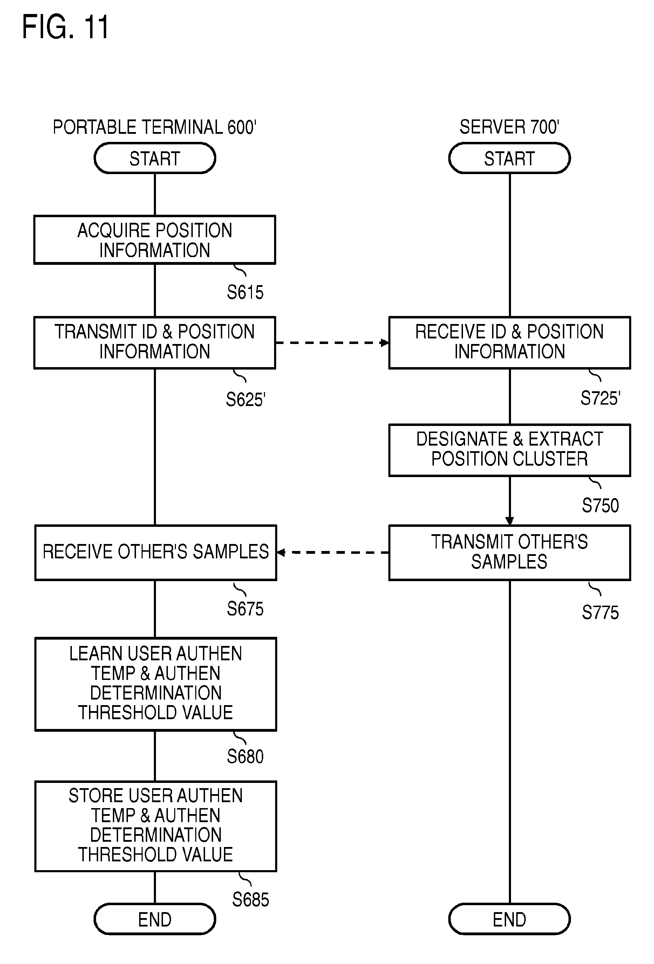

A user authentication template learning system according to a second embodiment will be described in detail with reference to Figs. 9, 10, and 11. Fig. 9 is a block diagram illustrating the configuration of a user authentication template learning system 2000 according to the embodiment. Fig. 10 is a flowchart illustrating an operation in sample registration of the user authentication template learning system 2000 according to the embodiment. Fig. 11 is a flowchart illustrating an operation in other's sample request of the user authentication template learning system 2000 according to the embodiment. The user authentication template learning system 2000 according to the embodiment includes the portable terminal 600' and the server 700'. The portable terminal 600' includes a sensor 605, a sample acquisition unit 610, a position information acquisition unit 615, a sample transmission unit 620, an other's sample request information transmission unit 625', a registration completion notification reception unit 640, an other's sample reception unit 675, a user authentication template learning unit 680, and a template storage unit 685. As the sensor 605 used in the embodiment, the pressure sensor array 105 described with reference to Fig. 3 or the fingerprint authentication sensor 205 described with reference to Fig. 4 may be used, as is the case with the first embodiment. Any device may be used as the sensor 605 as long as the device is capable of acquiring a sample which is used for biometric authentication and is small enough to be incorporated in a portable terminal. On the other hand, the server 700' includes a sample reception unit 720, an other's sample request information reception unit 725', a clustering unit 730', a sample storage unit 735, a registration completion notification transmission unit 740, a position cluster extraction unit 750, and an other's sample transmission unit 775.

An operation of the user authentication template learning system 2000 when the portable terminal 600' transmits a biometric authentication sample thereof to the server 700' so as to obtain registration of the biometric authentication sample will be first described. The sample acquisition unit 610 acquires a sample which is to be used for biometric authentication from the sensor 605 (S610). The position information acquisition unit 615 acquires current position information of the portable terminal 600' (S615). The position information acquisition unit 615 represents a function to acquire position information of the own terminal from a GPS satellite or a base station. The sample transmission unit 620 transmits the acquired sample with a user ID and the position information to the server 700' (S620). On the other hand, the sample reception unit 720 of the server 700' receives the sample with the user ID and the position information from the portable terminal 600' (S720). The clustering unit 730' classifies the received sample to any of two or more predetermined position clusters (S730'). The sample storage unit 735 stores the classified sample and the position cluster of the sample while associating the sample and the position cluster with the received user ID (S735). Accordingly, a feature cluster and time information illustrated in Fig. 5 are not used in this embodiment. When a sample is stored in the sample storage unit 735, the registration completion notification transmission unit 740 transmits registration completion notification to the portable terminal 600' (S740).

The registration completion notification reception unit 640 of the portable terminal 600' receives the registration completion notification from the server 700' (S640). Thus, each biometric authentication sample is classified and stored by using a position cluster which is defined in accordance with the position of the portable terminal 600' in the server 700', so that biometric authentication samples of which positions are close to each other are stored in the same position cluster (refer to a section of <Position cluster> for details).

Subsequently, an operation of the user authentication template learning system 2000 when the portable terminal 600' requests other's samples from the server 700', will be described. The methods (A) to (C), for example, may be set as a method by which the portable terminal 600' acquires other's samples from the server 700', as is the case with the first embodiment. For the sake of simplicity, description is given on the assumption that a trigger by which the portable terminal 600' requests other's samples from the server 700' is "reception of a user ID and position information by the server 700'" in any method.

In the case of the above-described other's sample acquisition method (A), all samples, which belong to a position cluster identical to the position cluster which is generated by the clustering unit 730' on the basis of the position information which is received by the sample reception unit 720 in registration (S720), other than the sample of the authentic user are extracted by the position cluster extraction unit 750 from the sample storage unit 735 (S750) so as to be transmitted as other's samples from the other's sample transmission unit 775 to the portable terminal 600' of which the user ID is received (S775). In the case of the other's sample acquisition method (B), the position information acquisition unit 615 of the portable terminal 600' acquires current position information of the portable terminal 600' after reception of registration completion notification (S615) and the other's sample request information transmission unit 625' transmits other's sample request information including the user ID and the position information to the server 700' (S625'). The other's sample request information reception unit 725' of the server 700' receives the other's sample request information including the user ID and the position information from the portable terminal 600' (S725'). As described above, reception operation triggers an other's sample transmission operation below.

The position cluster extraction unit 750 extracts all samples, which belong to a position cluster same as a position cluster corresponding to the user ID included in the received other's sample request information, other than a sample corresponding to the received user ID, namely, other than a sample of the authentic user, from the sample storage unit 735 (S750). The other's sample transmission unit 775 transmits the extracted samples as other's samples to the portable terminal 600' which has transmitted the user ID (S775). On the other hand, the other's sample reception unit 675 of the portable terminal 600' receives the other's samples from the server 700' (S675). The user authentication template learning unit 680 performs learning (re-learning) of the user authentication template and an authentication determination threshold value by using the other's samples and the sample of the authentic user (S680). The template storage unit 685 stores the learned user authentication template and authentication determination threshold value (S685).

Thus, in the user authentication template learning system 2000 of the embodiment, real other's samples of which current positions are close to a current position which has been registered on a server can be obtained so as to learn (re-learn) a user authentication template which has been learned and an authentication determination threshold value, enabling to locally optimize accuracy of biometric authentication. This is because security can be sufficiently ensured when a user authentication template is locally optimized on the basis of other's samples which are collected by narrowing down to positional relations in which malicious use may occur, since it is physically impossible for other people who are present in sufficiently far positions (other people who are present in Hokkaido when a user is present in Tokyo, for example) to pick up the portable terminal by accident or steal the portable terminal. Here, the case in which other's sample request information includes a user ID and position information has been described in this embodiment, but position information does not have to be included.

[Third embodiment]

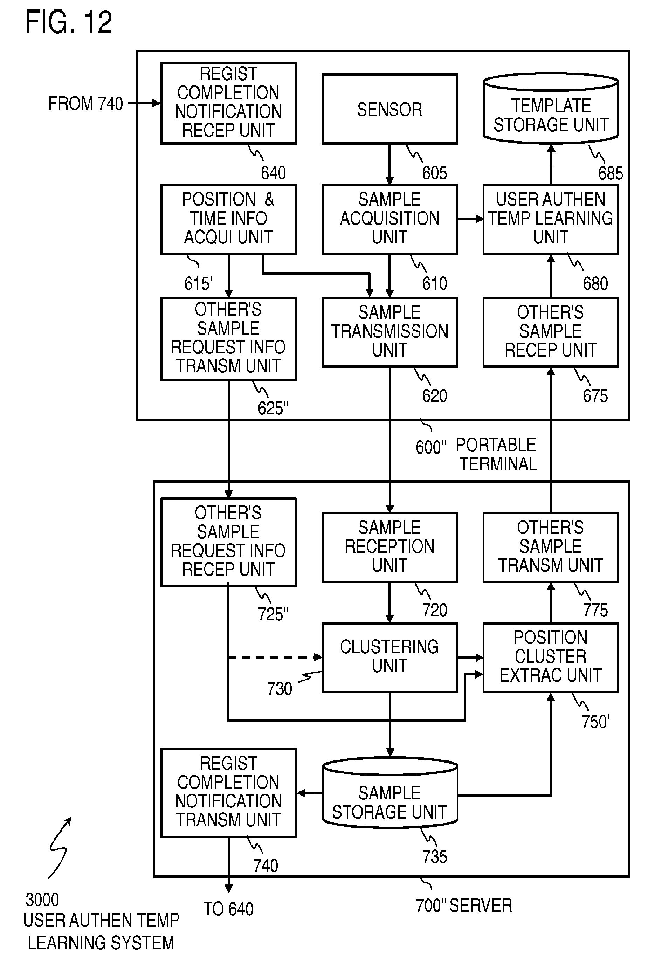

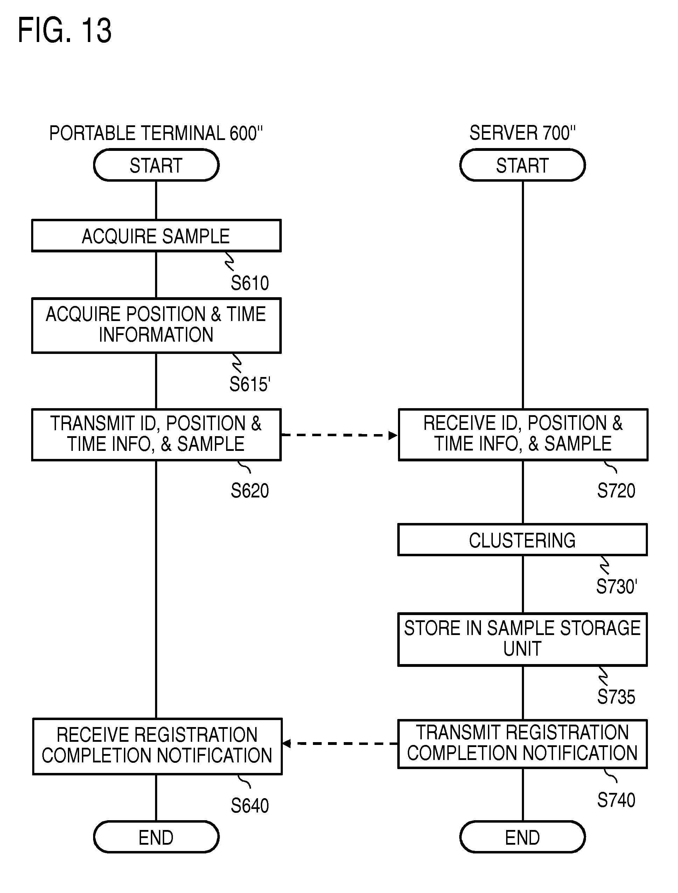

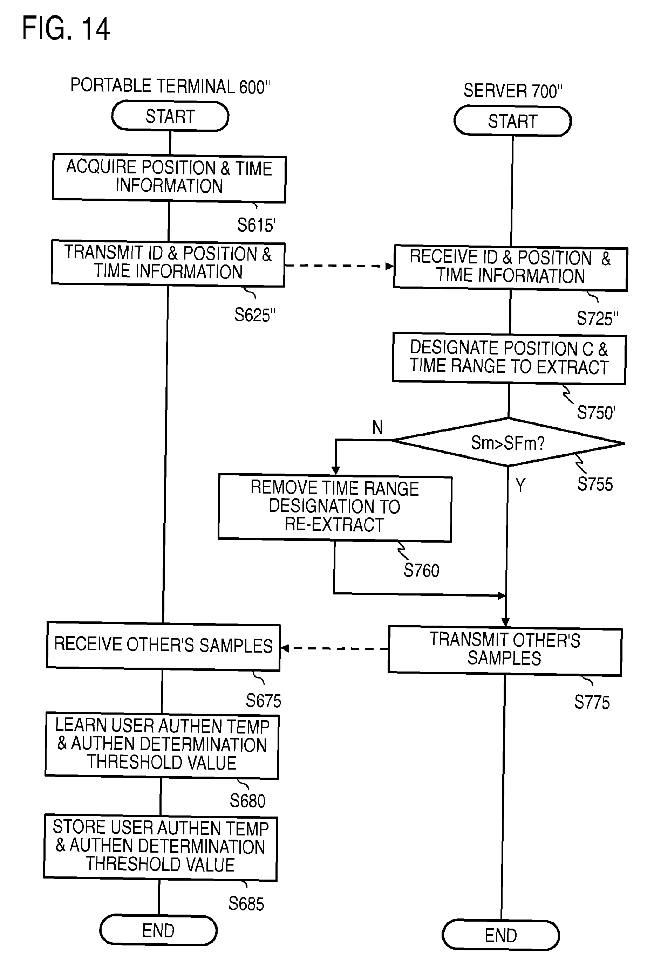

A user authentication template learning system according to a third embodiment will be described in detail with reference to Figs. 12, 13, and 14. Fig. 12 is a block diagram illustrating the configuration of a user authentication template learning system 3000 according to the embodiment. Fig. 13 is a flowchart illustrating an operation in sample registration of the user authentication template learning system 3000 according to the embodiment. Fig. 14 is a flowchart illustrating an operation in other's sample request of the user authentication template learning system 3000 according to the embodiment. The user authentication template learning system 3000 according to the embodiment includes the portable terminal 600" and the server 700". The portable terminal 600" includes a sensor 605, a sample acquisition unit 610, a position and time information acquisition unit 615', a sample transmission unit 620, an other's sample request information transmission unit 625", a registration completion notification reception unit 640, an other's sample reception unit 675, a user authentication template learning unit 680, and a template storage unit 685. As the sensor 605 used in the embodiment, the pressure sensor array 105 described with reference to Fig. 3 or the fingerprint authentication sensor 205 described with reference to Fig. 4 may be used, as is the case with the first embodiment. Any device may be used as the sensor 605 as long as the device is capable of acquiring a sample which is used for biometric authentication and is small enough to be incorporated in a portable terminal. On the other hand, the server 700" includes a sample reception unit 720, an other's sample request information reception unit 725", a clustering unit 730', a sample storage unit 735, a registration completion notification transmission unit 740, a position cluster extraction unit 750', and an other's sample transmission unit 775.

An operation of the user authentication template learning system 3000 when the portable terminal 600" transmits a biometric authentication sample thereof to the server 700" so as to obtain registration of the biometric authentication sample, will be first described. The sample acquisition unit 610 acquires a sample which is used for biometric authentication from the sensor 605 (S610). The position and time information acquisition unit 615' acquires current position information and time information of the portable terminal 600" (S615'). The position and time information acquisition unit 615' represents a function to acquire position information of the own terminal from a GPS satellite or a base station and to acquire time at which the portable terminal 600" acquires a sample. The sample transmission unit 620 transmits the acquired sample with a user ID and the position and time information to the server 700" (S620). On the other hand, the sample reception unit 720 of the server 700" receives the sample with the user ID and the position and time information from the portable terminal 600" (S720). The clustering unit 730' classifies the received samples to any of two or more predetermined position clusters (S730'). The sample storage unit 735 stores the classified sample and the position cluster of the sample while associating the sample and the position cluster with the received user ID and the received time information (S735). Accordingly, a feature cluster illustrated in Fig. 5 is not used in this embodiment. When a sample is stored in the sample storage unit 735, the registration completion notification transmission unit 740 transmits registration completion notification to the portable terminal 600" (S740). The registration completion notification reception unit 640 of the portable terminal 600" receives the registration completion notification from the server 700" (S640). Thus, biometric authentication samples are classified and stored by using a position cluster which is defined in accordance with the position of the portable terminal 600" in the server 700" when the portable terminal 600" obtains registration of a biometric authentication sample thereof. Therefore, biometric authentication samples of which positions are close to each other are stored in the same position cluster (refer to a section of <Position cluster> for details). Further, unlike the second embodiment, it should be noted that time information is also recorded in the sample storage unit 735.

Subsequently, an operation of the user authentication template learning system 3000 when the portable terminal 600" requests other's samples from the server 700" will be described. The methods (A) to (C), for example, may be set as a method by which the portable terminal 600" acquires other's samples from the server 700", as is the case with the first and second embodiments. For the sake of simplicity, description is given on the assumption that a trigger by which the portable terminal 600" requests other's samples from the server 700" is "reception of all of a user ID, position information, and time information by the server 700"" in any method.

In the case of the above-described other's sample acquisition method (A), all samples, which belong to a position cluster identical to the position cluster which is generated by the clustering unit 730' on the basis of the position information which is received by the sample reception unit 720 in registration (S720), other than the sample of the authentic user are extracted by the position cluster extraction unit 750 from the sample storage unit 735 (S750) so as to be transmitted as other's samples from the other's sample transmission unit 775 to the portable terminal 600' of which the user ID is received (S775). In the case of the other's sample acquisition method (B), the position and time information acquisition unit 615' of the portable terminal 600" acquires current position information and time information of the portable terminal 600" after reception of registration completion notification (S615') and the other's sample request information transmission unit 625" transmits other's sample request information including the user ID, the position information, and the time information to the server 700" (S625"). The other's sample request information reception unit 725" of the server 700" receives the other's sample request information including the user ID, the position information, and the time information from the portable terminal 600" (S725"). As described above, this reception operation triggers an other's sample transmission operation. The position cluster extraction unit 750' extracts samples, which belong to a position cluster identical to the position cluster corresponding to the user ID included in the received other's sample request information and belong to a time range predetermined with reference to the received time information, from the sample storage unit 735 (S750'). Samples to be extracted are limited to samples other than the sample of the authentic user corresponding to the received user ID.

Here, the number of other's samples required for learning (re-learning) of a user authentication template is denoted as SFm and the number of samples which are obtained by the extraction of step S750' is denoted as Sm. When Sm>SFm is not satisfied (S755N), the processing goes to step S760 and designation of the time range is removed to re-extract samples (S760). Then, the processing goes to step S775. On the other hand, when Sm>SFm is satisfied in step S755 (S755Y), the processing goes to step S775.

The other's sample transmission unit 775 transmits the extracted samples as other's samples to the portable terminal 600" which has transmitted the user ID (S775). On the other hand, the other's sample reception unit 675 of the portable terminal 600" receives the other's samples from the server 700" (S675). The user authentication template learning unit 680 performs learning (re-learning) of the user authentication template and an authentication determination threshold value by using the other's samples and the sample of the authentic user (S680). The template storage unit 685 stores the learned (re-learned) user authentication template and authentication determination threshold value (S685).

Thus, in the user authentication template learning system 3000 of the embodiment, real other's samples of which current positions are close to a current position which has been registered on a server and time information is also close to time information which has been registered on the server can be obtained so as to learn (re-learn) a user authentication template which has been learned, enabling to locally optimize accuracy of biometric authentication more optimally than the user authentication template learning system 2000 of the second embodiment. This is because security can be sufficiently ensured when a user authentication template is locally optimized on the basis of other's samples which are collected by narrowing down to positional and time relations in which malicious use may occur, since it is physically impossible to pick up the portable terminal by accident or steal the portable terminal when acquisition time is remarkably different, which represents absence in the same place on same time, even in a case of other's samples of an identical position cluster, though exhibiting the same sense as the second embodiment. Here, the case in which other's sample request information includes a user ID, position information, and time information has been described in this embodiment, but position information and time information do not have to be included.

[Fourth embodiment]

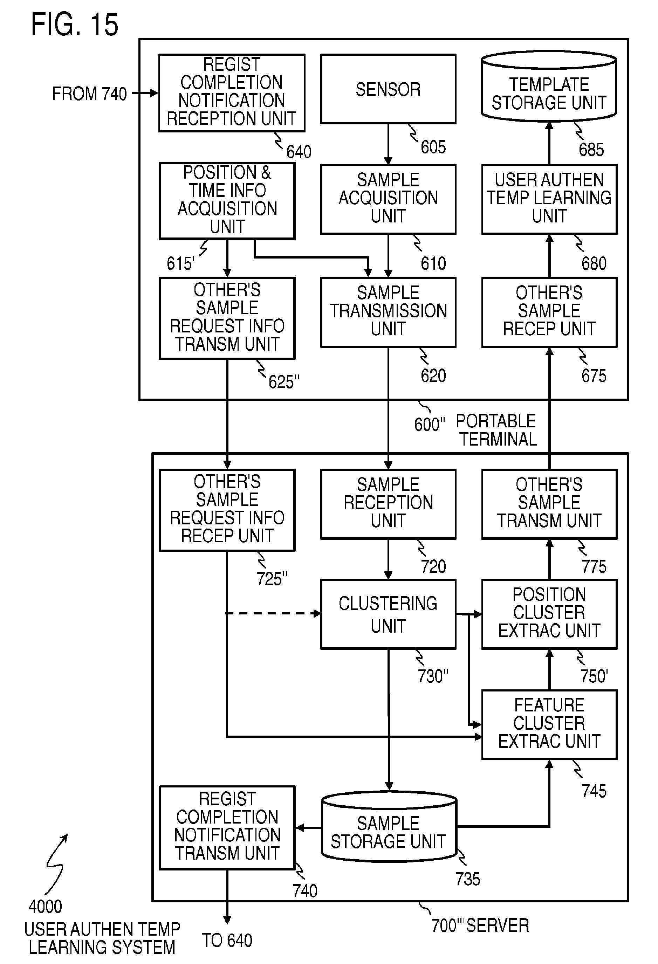

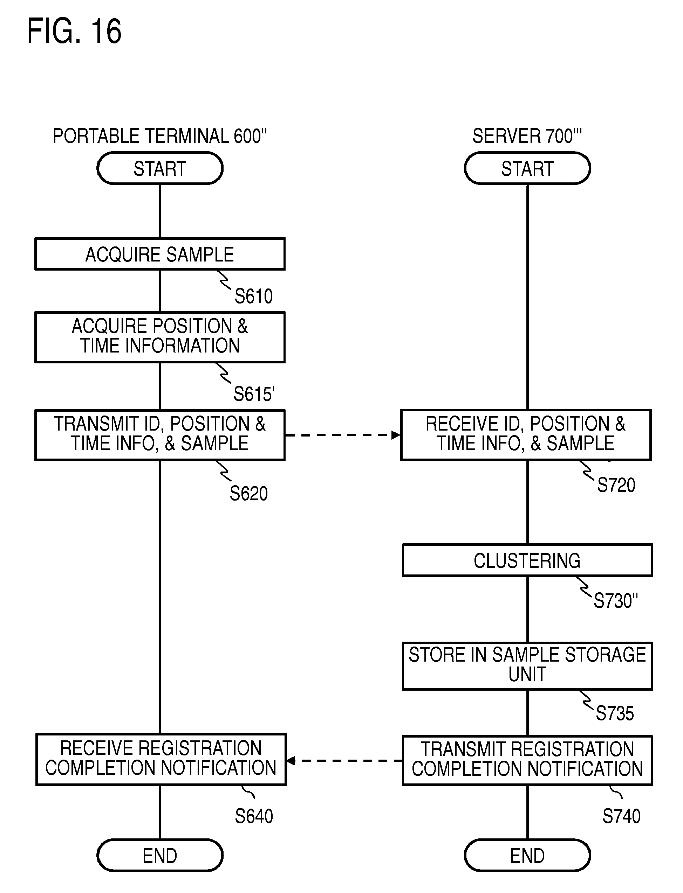

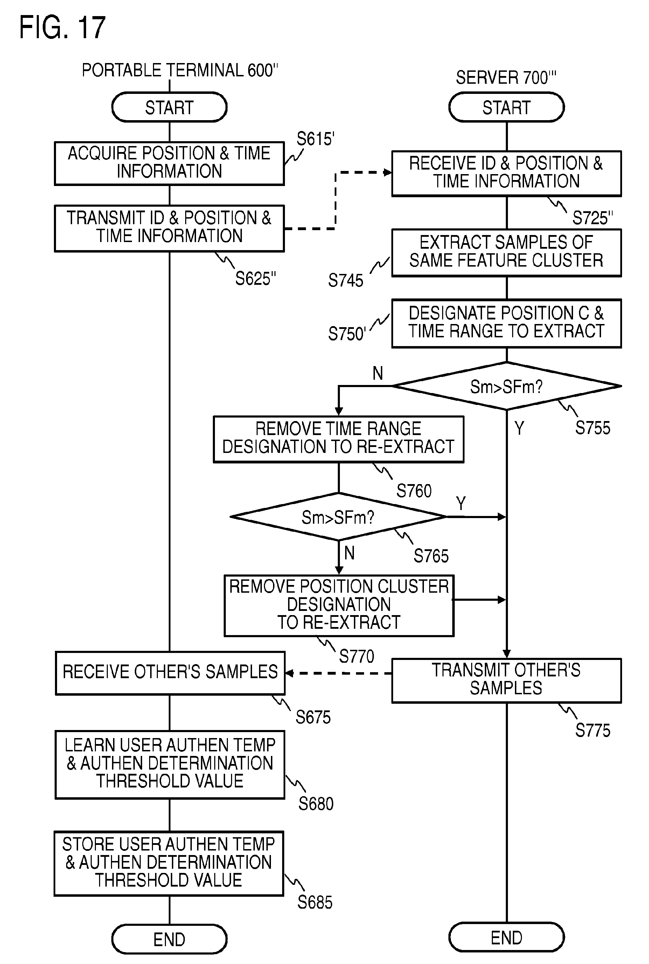

A user authentication template learning system according to a fourth embodiment will be described in detail with reference to Figs. 15, 16, and 17. Fig. 15 is a block diagram illustrating the configuration of a user authentication template learning system 4000 according to the embodiment. Fig. 16 is a flowchart illustrating an operation in sample registration of the user authentication template learning system 4000 according to the embodiment. Fig. 17 is a flowchart illustrating an operation in other's sample request of the user authentication template learning system 4000 according to the embodiment. The user authentication template learning system 4000 according to the embodiment includes the portable terminal 600" and the server 700"'. The portable terminal 600" has the same configuration as the portable terminal 600" of the third embodiment (Fig. 12), so that description of the configuration of the portable terminal 600" will be skipped. On the other hand, the server 700"' includes a sample reception unit 720, an other's sample request information reception unit 725", a clustering unit 730", a sample storage unit 735, a registration completion notification transmission unit 740, a feature cluster extraction unit 745, a position cluster extraction unit 750', and an other's sample transmission unit 775.

An operation of the user authentication template learning system 4000 when the portable terminal 600" transmits a biometric authentication sample thereof to the server 700"' so as to obtain registration of the biometric authentication sample, will be first described. The sample acquisition unit 610 acquires a sample which is used for biometric authentication from the sensor 605 (S610). The position and time information acquisition unit 615' acquires current position information and time information of the portable terminal 600" (S615'). The sample transmission unit 620 transmits the acquired sample with a user ID and the position and time information to the server 700"' (S620). On the other hand, the sample reception unit 720 of the server 700"' receives the sample with the user ID and the position and time information from the portable terminal 600" (S720). The clustering unit 730" classifies the received sample to any one of two or more predetermined position clusters and classifies the received sample to any one of two or more predetermined feature clusters (S730"). The sample storage unit 735 stores the classified samples, the feature cluster, and the position cluster of the samples while associating the samples, the feature cluster, and the position cluster with the received user ID and the received time information (S735). Accordingly, all pieces of information illustrated in Fig. 5 are used in this embodiment. The registration completion notification transmission unit 740 transmits registration completion notification to the portable terminal 600" (S740). The registration completion notification reception unit 640 of the portable terminal 600" receives the registration completion notification from the server 700"' (S640). Thus, biometric authentication samples are classified and stored by using position clusters which are defined in accordance with the position of the portable terminal 600" in the server 700"' and feature clusters which are defined in accordance with features of the biometric authentication samples when the portable terminal 600" obtains registration of the biometric authentication thereof. Therefore, the biometric authentication samples of which positions are close to each other are stored in the same position cluster and the biometric authentication samples of which features are similar to each other are recorded in the same feature cluster. Further, unlike the second embodiment, it should be noted that time information is also recorded in the sample storage unit 735.

Subsequently, an operation of the user authentication template learning system 4000 when the portable terminal 600" requests other's samples from the server 700", will be described. The methods (A) to (C), for example, may be set as a method by which the portable terminal 600" acquires other's samples from the server 700"', as is the case with the first, second, and third embodiments. For the sake of simplicity, description is given on the assumption that a trigger by which the portable terminal 600" requests other's samples from the server 700"' is "reception of all of a user ID, position information, and time information by the server 700"' in any method.

In the case of the above-described other's sample acquisition method (A), all samples, which belong to a position cluster identical to the position cluster which is generated by the clustering unit 730' on the basis of the position information which is received by the sample reception unit 720 in registration (S720), other than the sample of the authentic user are extracted by the position cluster extraction unit 750 from the sample storage unit 735 (S750) so as to be transmitted as other's samples from the other's sample transmission unit 775 to the portable terminal 600" of which the user ID is received (S775). In the case of the other's sample acquisition method (B), the position and time information acquisition unit 615' of the portable terminal 600" acquires current position information and time information of the portable terminal 600" after reception of registration completion notification (S615') and the other's sample request information transmission unit 625 "transmits other's sample request information including the user ID, the position information, and the time information to the server 700"' (S625"). The other's sample request information reception unit 725" of the server 700"' receives the other's sample request information including the user ID, the position information, and the time information from the portable terminal 600" (S725"). As described above, this reception operation triggers an other's sample transmission operation. The feature cluster extraction unit 745 extracts all the samples, which belong to a feature cluster identical to the feature cluster which corresponds to the user ID included in the received other's sample request information, other than the sample of the authentic user, from the sample storage unit 735 (S745). The position cluster extraction  unit 750' further extracts all the samples, which belong to a position cluster identical to the position cluster corresponding to the received user ID and belong to a time range predetermined with reference to the received time information, from the samples which are extracted by the feature cluster extraction unit 745 (S750').

Here, when Sm>SFm described above is not satisfied (S755N), the processing goes to step S760 and designation of the time range is removed to re-extract samples (S760). When Sm>SFm is not satisfied even by the removal of the time range designation of step S760 (S765N), extraction designating a position cluster is removed to re-extract samples (S770) and the processing goes to step S775. On the other hand, when Sm>SFm is satisfied in step S755 and step S765 (S755Y, S765Y), the processing goes to step S775.

The other's sample transmission unit 775 transmits the extracted samples as other's samples to the portable terminal 600" which has transmitted the user ID (S775). On the other hand, the other's sample reception unit 675 of the portable terminal 600" receives the other's samples from the server 700"' (S675). The user authentication template learning unit 680 performs learning of the user authentication template and an authentication determination threshold value by using the other's samples and the sample of the authentic user (S680). The template storage unit 685 stores the learned user authentication template and authentication determination threshold value (S685).

Thus, in the user authentication template learning system 4000 of the embodiment, real other's samples of which features are similar to the sample of the authentic user which has been registered on a server 700"', further, of which current positions are close to that of the sample of the authentic user, and of which time information is also close to that of the sample of the authentic user can be obtained so as to learn (re-learn) a user authentication template which has been learned and an authentication determination threshold value, enabling to improve and locally optimize accuracy of biometric authentication. Here, the case in which other's sample request information includes a user ID, position information, and time information has been described in this embodiment, but position information and time information do not have to be included.

Each type of processing described above may be executed not only time sequentially according to the order in the description but also in parallel or individually when necessary or according to the processing capability of each apparatus that executes the processing. Appropriate changes can be made to the present invention without departing from the scope of the present invention.

When the configurations described above are implemented by a computer, the processing details of the functions that should be provided by each apparatus are described in a program. When the program is executed by the computer, the processing functions are implemented on the computer.

The program containing the processing details may be recorded in a computer-readable recording medium. The computer-readable recording medium may be any type of medium, such as a magnetic recording device, an optical disc, a magneto-optical recording medium, or a semiconductor memory.

The program may distributed by selling, transferring, or lending a portable recording medium, such as a DVD or a CD-ROM, with the program recorded on it, for example. The program may also be distributed by storing the program in a storage unit of a server computer and transferring the program from the server computer to another computer through a network.

A computer that executes this type of program first stores the program recorded on a portable recording medium or the program transferred from the server computer in its storage unit. Then, the computer reads the program stored in its storage unit and executes processing in accordance with the read program. In a different program execution form, the computer may read the program directly from the portable recording medium and execute processing in accordance with the program, or the computer may execute processing in accordance with the program each time the computer receives the program transferred from the server computer. Alternatively, the above-described processing may be executed by a so-called application service provider (ASP) service, in which the processing functions are implemented just by giving program execution instructions and obtaining the results without transferring the program from the server computer to the computer. The program of this form includes information that is provided for use in processing by the computer and is treated correspondingly as a program (something that is not a direct instruction to the computer but is data or the like that has characteristics that determine the processing executed by the computer).

In the description given above, each apparatus is implemented by executing the predetermined program on the computer, but at least a part of the processing may be implemented by hardware.

**Claims**

1. A user authentication template learning system including two or more mobile information terminals and a server; wherein
   each mobile information terminal comprises:

   a sample acquisition unit configured to acquire a sample of an authentic user, the sample being used for biometric authentication;
   a sample transmission unit configured to transmit the acquired sample of the authentic user along with a user ID for specifying the mobile information terminal to the server;
   an other's sample reception unit configured to receive other's samples from the server;
   a user authentication template learning unit configured to perform learning of a user authentication template and an authentication determination threshold value by using the other's samples and samples of the authentic user; and
   a template storage unit configured to store the user authentication template and the authentication determination threshold value that are learned; and

   the server comprises:

   a sample reception unit configured to receive a sample and a user ID from each mobile information terminal;
   a clustering unit configured to classify each sample that is received into any one of two or more predetermined feature clusters;
   a sample storage unit configured to store the sample that is classified and the feature cluster of the sample in association with the user ID that is received;
   a feature cluster extraction unit configured to extract all samples belonging to a feature cluster identical to the feature cluster corresponding to the user ID that is received, other than the sample corresponding to the user ID that is received, from the sample storage unit; and
   an other's sample transmission unit configured to transmit the samples that are extracted, as other's samples, to the mobile information terminal that has transmitted the user ID.

2. The user authentication template learning system according to claim 1, wherein,
   each mobile information terminal further comprises a position and time information acquisition unit configured to acquire current position information and time information of the mobile information terminals;

the sample transmission unit is configured to transmit the acquired sample along with the position information, the time information, and the user ID to the server;

the sample reception unit of the server is configured to receive the sample along with the position information, the time information, and the user ID from the mobile information terminal;

the sample storage unit of the server is configured to store the sample that is classified, a position cluster, and the feature cluster in association with the user ID that is received and the time information that is received; and

the server further comprises a position cluster extraction unit configured to extract all samples belonging to a position cluster identical to the position cluster corresponding to the user ID that is received and belonging to a time range predetermined with reference to the time information that is received, as the other's samples from the samples that are extracted by the feature cluster extraction unit.

3. A user authentication template learning system including two or more mobile information terminals and a server; wherein,

each mobile information terminal comprises:

a sample acquisition unit configured to acquire a sample of an authentic user, the sample being used for biometric authentication;

a position information acquisition unit configured to acquire current position information of the mobile information terminal;

a sample transmission unit configured to transmit the acquired sample of the authentic user along with the position information and a user ID for specifying the mobile information terminals to the server;

an other's sample reception unit configured to receive other's samples from the server;

a user authentication template learning unit configured to perform learning of a user authentication template and an authentication determination threshold value by using the other's samples and samples of the authentic user; and

a template storage unit configured to store the user authentication template and the authentication determination threshold value that are learned; and

the server comprises:

a sample reception unit configured to receive a sample along with position information and a user ID from each mobile information terminal;

a clustering unit configured to classify each sample that is received into any one of two or more predetermined position clusters on the basis of the position information that is received;

a sample storage unit configured to store the sample that is classified and the position cluster in association with the user ID that is received;

a position cluster extraction unit configured to extract all samples belonging to a position cluster identical to the position cluster corresponding to the user ID that is received, other than the sample corresponding to the user ID that is received, from the sample storage unit; and

an other's sample transmission unit configured to transmit the samples that are extracted, as other's samples, to the mobile information terminal that has transmitted the user ID.

4. The user authentication template learning system according to claim 2, wherein,

each mobile information terminal further comprises a time information acquisition unit configured to acquire current time information of the mobile information terminal;

the sample transmission unit is configured to transmit the acquired sample along with the position information, the time information, and the user ID to the server;

the sample reception unit of the server is configured to receive the sample along with the position information, the time information, and the user ID from the mobile information terminal;

the sample storage unit is configured to store the sample that is classified and the position cluster in a association with the user ID that is received and the time information that is received; and

the position cluster extraction unit is configured to extract all samples belonging to a position cluster identical to the position cluster corresponding to the user ID that is received and belonging to a time range predetermined with reference to the time information that is received, other than the sample corresponding to the user ID that is received, as the other's samples from the sample storage unit.

5. The user authentication template learning system according to any one of claim 1 to claim 4, wherein each mobile information terminal further comprises an other's sample request information transmission unit configured to transmit other's sample request information that includes the user ID to the server, and the server further comprises an other's sample request information reception unit configured to receive other's sample request information that includes the user ID.

6. A user authentication template learning method in which two or more mobile information terminals and a server are used, comprising:

a sample acquisition step in which each mobile information terminal acquires a sample of an authentic user, the sample being used for biometric authentication;

a sample transmission step in which the mobile information terminal transmits the acquired sample of the authentic user along with a user ID for specifying the mobile information terminal to the server;

a sample reception step in which the server receives the sample and the user ID from the mobile information terminal;

a clustering step in which the server classifies the sample that is received into any one of two or more predetermined feature clusters;

a sample storage step in which the server stores the sample that is classified and the feature cluster of the sample in association with the user ID that is received;

a feature cluster extraction step in which the server extracts all samples belonging to a feature cluster identical to the feature cluster corresponding to the user ID that is received, other than the sample corresponding to the user ID that is received, from samples that are stored in the sample storage step;

an other's sample transmission step in which the server transmits the samples that are extracted, as other's samples, to the mobile information terminal that has transmitted the user ID;

an other's sample reception step in which the mobile information terminal receives other's samples from the server;

a user authentication template learning step in which the mobile information terminal performs learning of a user authentication template and an authentication determination threshold value by using the other's samples and samples of the authentic user; and

a template storage step in which the mobile information terminals stores the user authentication template and the authentication determination threshold value that are learned.

7. The user authentication template learning method according to claim 6, further comprising:

a position and time information acquisition step in which the mobile information terminal acquires current position information and time information of the mobile information terminal; wherein,

in the sample transmission step, the mobile information terminal transmits the acquired sample along with the position information, the time information, and the user ID to the server;

in the sample reception step performed by the server, the sample is received along with the position information, the time information, and the user ID from the mobile information terminals;

in the clustering step performed by the server, the sample is classified into any one of two or more predetermined position clusters on the basis of the position information that is received;

in the sample storage step performed by the server, the sample that is classified, the position cluster, and the feature cluster are stored in association with the user ID that is received and the time information that is received; and

the learning method further includes a position cluster extraction step in which the server extracts all samples belonging to a position cluster identical to the position cluster corresponding to the user ID that is received and belonging to a time range predetermined with reference to the time information that is received, as the other's samples from the samples that are extracted in the feature cluster extraction step.

8. A user authentication template learning method in which two or more mobile information terminals and a server are used, comprising:

a sample acquisition step in which each mobile information terminal acquires a sample of an authentic user, the sample being used for biometric authentication;

a position information acquisition step in which the mobile information terminal acquires current position information of the mobile information terminal;

a sample transmission step in which the mobile information terminal transmits the acquired sample of the authentic user along with the position information and a user ID for specifying the mobile information terminal to the server;

a sample reception step in which the server receives the sample along with the position information and the user ID from the mobile information terminal;

a clustering step in which the server classifies the sample that is received into any one of two or more predetermined position clusters on the basis of the position information that is received;

a sample storage step in which the server stores the sample that is classified and the position cluster in association with the user ID that is received;

a position cluster extraction step in which the server extracts all samples belonging to a position cluster identical to the position cluster corresponding to the user ID that is received, other than the sample corresponding to the user ID that is received, from samples that are stored in the sample storage step;

an other's sample transmission step in which the server transmits the samples that are extracted, as other's samples, to the mobile information terminal that has transmitted the user ID;

an other's sample reception step in which the mobile information terminal receives the other's samples from the server;

a user authentication template learning step in which the mobile information terminal performs learning of a user authentication template and an authentication determination threshold value by using the other's samples and samples of the authentic user; and

a template storage step in which the mobile information terminal stores the user authentication template and the authentication determination threshold value that are learned.

9. The user authentication template learning method according to claim 8, further comprising:

a time information acquisition step in which the mobile information terminal acquires current time information of the mobile information terminal; wherein,

in the sample transmission step performed by the mobile information terminal, the acquired sample is transmitted along with the position information, the time information, and the user ID for specifying the mobile information terminal to the server;

in the sample reception step performed by the server, the sample is received along with the position information, the time information, and the user ID from the mobile information terminal;

in the sample storage step performed by the server, the sample that is classified and the position cluster are stored in association with the user ID that is received and the time information that is received; and

in the position cluster extraction step performed by the server, all samples belonging to a position cluster identical to the position cluster corresponding to the user ID that is received and belonging to a time range predetermined with reference to the time information that is received, other than the sample corresponding to the user ID that is received, are extracted as the other's samples from the samples that are stored in the sample storage step.

10. The user authentication template learning method according to any one of claim 6 to claim 9, further comprising:

an other's sample request information transmission step in which the mobile information terminal transmits other's sample request information that includes the user ID to the server; and

an other's sample request information reception step in which the server receives other's sample request information that includes the user ID.

11. A recording medium that can be read by a computer in which a program for executing the user authentication template learning method according to any one of claim 6 to claim 9 is recorded.

# FIG. 1A

○: SAMPLE FROM PERSON A
●: TEMPLATE OF PERSON A

△: SAMPLE FROM AUTHENTIC USER
▲: TEMPLATE OF AUTHENTIC USER

# FIG. 1B

○: SAMPLE FROM PERSON A
●: TEMPLATE OF PERSON A

△: SAMPLE FROM AUTHENTIC USER
▲: TEMPLATE OF AUTHENTIC USER

□: SAMPLE FROM PERSON B
■: TEMPLATE OF PERSON B

# FIG. 1C

✧: SAMPLE FROM PERSON C
✚: TEMPLATE OF PERSON C

# FIG. 2

16 LIQUID CRYSTAL DISPLAY

11 KEY ARRANGED FACE

LEFT SIDE FACE 12

15
BOTTOM FACE

600 PORTABLE TERMINAL
(600')
(600'')
(600''')

# FIG. 3

11 KEY ARRANGED FACE

LEFT SIDE FACE 12

13 RIGHT SIDE FACE

PRESSURE SENSOR ARRAY 105

14 REAR FACE

15 BOTTOM FACE

600(600',600'',600''')  PORTABLE TERMINAL

LIQUID CRYSTAL DISPLAY

BOTTOM FACE

MEASUREMENT RESULT OF GRIPPING-PRESSURE DISTRIBUTION ON FACE 12

MEASUREMENT RESULT OF GRIPPING-PRESSURE DISTRIBUTION ON FACE 14

MEASUREMENT RESULT OF GRIPPING-PRESSURE DISTRIBUTION ON FACE 13

# FIG. 4

16 LIQUID CRYSTAL DISPLAY

11 KEY ARRANGED FACE

LEFT SIDE FACE 12

FINGERPRINT
AUTHENTICATION
SENSOR 205

15
BOTTOM FACE

600
(600')
(600")
(600"')   PORTABLE TERMINAL

# FIG. 5

| SN | USER ID | FEATURE CLUSTER | SAMPLE X | POSITION CLUSTER | TIME INFORMATION |
|---|---|---|---|---|---|
| 1 | AWE4- - -SRGS | 09 | $X_1$ | 28-04 | 11-02-26-18 |
| 2 | S1DT- - -52SD | 52 | $X_2$ | 23-01 | 11-02-24-08 |
| 3 | YG46- - -4A1E | 23 | $X_3$ | 12-07 | 11-02-25-19 |
| 4 | 9A6A- - -YA34 | 41 | $X_4$ | 13-07 | 11-02-25-12 |
| 5 | 4JD6- - -F78A | 65 | $X_5$ | 26-01 | 11-02-26-10 |
| 6 | 36YE- - -AWH1 | 81 | $X_6$ | 40-02 | 11-02-25-09 |
| 7 | 7ER3- - -984E | 53 | $X_7$ | 16-05 | 11-02-23-18 |
| 8 | 56RT- - -623E | 22 | $X_8$ | 23-01 | 11-02-24-08 |
| 9 | 79SE- - -GEHS | 09 | $X_9$ | 27-08 | 11-02-23-22 |
| 10 | 36ST- - -R56A | 04 | $X_{10}$ | 26-01 | 11-02-24-23 |
| 11 | ERH6- - -69E5 | 15 | $X_{11}$ | 27-01 | 11-02-25-15 |
| 12 | QJT0- - -53ZU | 16 | $X_{12}$ | 12-01 | 11-02-25-10 |
| 13 | U3BA- - -A932 | 84 | $X_{13}$ | 28-06 | 11-02-26-19 |
| 14 | J6KS- - -6E9A | 72 | $X_{14}$ | 28-04 | 11-02-26-18 |
| 15 | 12SE- - -E95R | 63 | $X_{15}$ | 13-01 | 11-02-24-13 |
| 16 | 4E7R- - -5AQ5 | 35 | $X_{16}$ | 15-07 | 11-02-25-17 |
| 17 | 6TRU- - -6FGZ | 91 | $X_{17}$ | 11-08 | 11-02-23-10 |
| … | … | … | … | … | … |

# FIG. 6

FROM 740 →

**600 PORTABLE TERMINAL**

- REGIST COMPLETION NOTIFICATION RECEP UNIT — 640
- SENSOR — 605
- TEMPLATE STORAGE UNIT — 685
- SAMPLE ACQUISITION UNIT — 610
- USER AUTHEN TEMPLATE LEARNING UNIT — 680
- OTHER'S SAMPLE REQUEST INFO TRANSM UNIT — 625
- SAMPLE TRANSMISSION UNIT — 620
- OTHER'S SAMPLE RECEP UNIT — 675

**700 SERVER**

- OTHER'S SAMPLE REQUEST INFO RECEP UNIT — 725
- SAMPLE RECEP UNIT — 720
- OTHER'S SAMPLE TRANSM UNIT — 775
- CLUSTERING UNIT — 730
- FEATURE CLUSTER EXTRAC UNIT — 745
- REGIST COMPLETION NOTIFICATION TRANSM UNIT — 740
- SAMPLE STORAGE UNIT — 735

TO 640

1000 USER AUTHEN TEMP LEARNING SYSTEM

# FIG. 7

PORTABLE TERMINAL 600

START

ACQUIRE SAMPLE
S610

TRANSMIT SAMPLE - - - → RECEIVE SAMPLE
S620                          S720

CLUSTERING
S730

STORE IN SAMPLE
STORAGE UNIT
S735

RECEIVE REGISTRATION ← - - - TRANSMIT REGISTRATION
COMPLETION NOTIFICATION        COMPLETION NOTIFICATION
S640                                      S740

END

SERVER 700

START

END

# FIG. 8

PORTABLE TERMINAL 600

( START )

SERVER 700

( START )

| TRANSMIT ID INFORMATION | - - - - - ▶ | RECEIVE ID INFORMATION |

S620 (S625)

S720 (S725)

EXTRACT SAMPLES OF
SAME FEATURE CLUSTER

S745

RECEIVE OTHER'S SAMPLES ◀ - - - - - TRANSMIT OTHER'S
SAMPLES

S675

S775

LEARN USER AUTHEN TEMP
& AUTHEN DETERMINATION
THRESHOLD VALUE

S680

STORE USER AUTHEN TEMP
& AUTHEN DETERMINATION
THRESHOLD VALUE

S685

( END )

( END )

## FIG. 9

FROM 740 → REGIST COMPLETION NOTIFICATION RECEP UNIT
640

POSITION INFORMATION ACQUI UNIT
615

OTHER'S SAMPLE REQUEST INFO TRANSM UNIT
625'

SENSOR
605

SAMPLE ACQUISITION UNIT
610

SAMPLE TRANSM UNIT
620

TEMPLATE STORAGE UNIT
685

USER AUTHEN TEMP LEARNING UNIT
680

OTHER'S SAMPLE RECEP UNIT
675

600' PORTABLE TERMINAL

OTHER'S SAMPLE REQUEST INFO RECEP UNIT
725'

SAMPLE RECEPTION UNIT
720

OTHER'S SAMPLE TRANSM UNIT
775

CLUSTERING UNIT
730'

POSITION CLUSTER EXTRAC UNIT
750

REGIST COMPLETION NOTIFICATION TRANSM UNIT
740

SAMPLE STORAGE UNIT
735

2000

USER AUTHEN TEMP LEARNING SYSTEM

TO 640

700' SERVER

FIG. 10

PORTABLE
TERMINAL 600'

START

ACQUIRE SAMPLE
S610

ACQUIRE POSITION
INFORMATION
S615

TRANSMIT ID, POSITION
INFORMATION, & SAMPLE
S620

RECEIVE REGISTRATION
COMPLETION NOTIFICATION
S640

END

SERVER 700'

START

RECEIVE ID, POSITION
INFORMATION, & SAMPLE
S720

CLUSTERING
S730'

STORE IN SAMPLE STORAGE
UNIT
S735

TRANSMIT REGISTRATION
COMPLETION NOTIFICATION
S740

END

# FIG. 11

**PORTABLE TERMINAL 600'**

START

ACQUIRE POSITION INFORMATION

S615

TRANSMIT ID & POSITION INFORMATION

S625'

RECEIVE OTHER'S SAMPLES

S675

LEARN USER AUTHEN TEMP & AUTHEN DETERMINATION THRESHOLD VALUE

S680

STORE USER AUTHEN TEMP & AUTHEN DETERMINATION THRESHOLD VALUE

S685

END

**SERVER 700'**

START

RECEIVE ID & POSITION INFORMATION

S725'

DESIGNATE & EXTRACT POSITION CLUSTER

S750

TRANSMIT OTHER'S SAMPLES

S775

END

## FIG. 12

**600" PORTABLE TERMINAL**

| | | |
|---|---|---|
| REGIST COMPLETION NOTIFICATION RECEP UNIT — 640 | SENSOR — 605 | TEMPLATE STORAGE UNIT — 685 |
| POSITION & TIME INFO ACQUI UNIT — 615' | SAMPLE ACQUISITION UNIT — 610 | USER AUTHEN TEMP LEARNING UNIT — 680 |
| OTHER'S SAMPLE REQUEST INFO TRANSM UNIT — 625" | SAMPLE TRANSMISSION UNIT — 620 | OTHER'S SAMPLE RECEP UNIT — 675 |

FROM 740 → 640

**700" SERVER**

| | | |
|---|---|---|
| OTHER'S SAMPLE REQUEST INFO RECEP UNIT — 725" | SAMPLE RECEPTION UNIT — 720 | OTHER'S SAMPLE TRANSM UNIT — 775 |
| | CLUSTERING UNIT — 730' | POSITION CLUSTER EXTRAC UNIT — 750' |
| REGIST COMPLETION NOTIFICATION TRANSM UNIT — 740 | SAMPLE STORAGE UNIT — 735 | |

TO 640

3000 USER AUTHEN TEMP LEARNING SYSTEM

# FIG. 13

PORTABLE TERMINAL 600"

SERVER 700"

```
( START )                          ( START )
    │                                  │
┌───────────────────────┐              │
│   ACQUIRE SAMPLE       │             │
└───────────────────────┘             │
    │         S610                      │
┌───────────────────────┐              │
│ ACQUIRE POSITION & TIME│             │
│    INFORMATION         │             │
└───────────────────────┘             │
    │         S615'                     │
┌───────────────────────┐    ┌───────────────────────┐
│  TRANSMIT ID, POSITION &│ --▶│ RECEIVE ID, POSITION &│
│   TIME INFO, & SAMPLE  │    │   TIME INFO, & SAMPLE │
└───────────────────────┘    └───────────────────────┘
    │         S620                      │         S720
                              ┌───────────────────────┐
                              │      CLUSTERING        │
                              └───────────────────────┘
                                        │         S730'
                              ┌───────────────────────┐
                              │ STORE IN SAMPLE STORAGE│
                              │         UNIT           │
                              └───────────────────────┘
                                        │         S735
┌───────────────────────┐    ┌───────────────────────┐
│  RECEIVE REGISTRATION  │ ◀--│ TRANSMIT REGISTRATION  │
│ COMPLETION NOTIFICATION│    │ COMPLETION NOTIFICATION│
└───────────────────────┘    └───────────────────────┘
    │         S640                      │         S740
( END )                            ( END )
```

# FIG. 14

PORTABLE TERMINAL 600"

START

ACQUIRE POSITION & TIME INFORMATION

S615'

TRANSMIT ID & POSITION & TIME INFORMATION

S625"

RECEIVE OTHER'S SAMPLES

S675

LEARN USER AUTHEN TEMP & AUTHEN DETERMINATION THRESHOLD VALUE

S680

STORE USER AUTHEN TEMP & AUTHEN DETERMINATION THRESHOLD VALUE

S685

END

SERVER 700"

START

RECEIVE ID & POSITION & TIME INFORMATION

S725"

DESIGNATE POSITION C & TIME RANGE TO EXTRACT

S750'

$Sm > SFm?$

N

REMOVE TIME RANGE DESIGNATION TO RE-EXTRACT

S760

S755

Y

TRANSMIT OTHER'S SAMPLES

S775

END

## FIG. 15

FROM 740 →

REGIST COMPLETION NOTIFICATION RECEPTION UNIT
640

SENSOR
605

TEMPLATE STORAGE UNIT
685

POSITION & TIME INFO ACQUISITION UNIT
615'

SAMPLE ACQUISITION UNIT
610

USER AUTHEN TEMP LEARNING UNIT
680

OTHER'S SAMPLE REQUEST INFO TRANSM UNIT
625"

SAMPLE TRANSMISSION UNIT
620

OTHER'S SAMPLE RECEP UNIT
675

600" PORTABLE TERMINAL

OTHER'S SAMPLE REQUEST INFO RECEP UNIT
725"

SAMPLE RECEPTION UNIT
720

OTHER'S SAMPLE TRANSM UNIT
775

CLUSTERING UNIT
730"

POSITION CLUSTER EXTRAC UNIT
750'

FEATURE CLUSTER EXTRAC UNIT
745

REGIST COMPLETION NOTIFICATION TRANSM UNIT
740

SAMPLE STORAGE UNIT
735

4000
USER AUTHEN TEMP LEARNING SYSTEM

TO 640

700'" SERVER

33

# FIG. 16

PORTABLE TERMINAL 600"

START

ACQUIRE SAMPLE
S610

ACQUIRE POSITION &
TIME INFORMATION
S615'

TRANSMIT ID, POSITION &
TIME INFO, & SAMPLE
S620

RECEIVE REGISTRATION
COMPLETION NOTIFICATION
S640

END

SERVER 700'''

START

RECEIVE ID, POSITION &
TIME INFO, & SAMPLE
S720

CLUSTERING
S730"

STORE IN SAMPLE STORAGE
UNIT
S735

TRANSMIT REGISTRATION
COMPLETION NOTIFICATION
S740

END

# FIG. 17

PORTABLE TERMINAL 600''

START

ACQUIRE POSITION & TIME INFORMATION — S615'

TRANSMIT ID & POSITION & TIME INFORMATION — S625''

RECEIVE OTHER'S SAMPLES — S675

LEARN USER AUTHEN TEMP & AUTHEN DETERMINATION THRESHOLD VALUE — S680

STORE USER AUTHEN TEMP & AUTHEN DETERMINATION THRESHOLD VALUE — S685

END

SERVER 700''''

START

RECEIVE ID & POSITION & TIME INFORMATION — S725''

EXTRACT SAMPLES OF SAME FEATURE CLUSTER — S745

DESIGNATE POSITION C & TIME RANGE TO EXTRACT — S750'

Sm>SFm? — S755
N / Y

REMOVE TIME RANGE DESIGNATION TO RE-EXTRACT — S760

Sm>SFm? — S765
Y / N

REMOVE POSITION CLUSTER DESIGNATION TO RE-EXTRACT — S770

TRANSMIT OTHER'S SAMPLES — S775

END

## INTERNATIONAL SEARCH REPORT

| International application No. |
|---|
| PCT/JP2012/054754 |

| A. CLASSIFICATION OF SUBJECT MATTER |
|---|
| *G06N3/00*(2006.01)i, *A61B5/117*(2006.01)i, *G06F21/20*(2006.01)i, *G06T7/00* (2006.01)i, *H04L9/32*(2006.01)i |

According to International Patent Classification (IPC) or to both national classification and IPC

| B. FIELDS SEARCHED |
|---|
| Minimum documentation searched (classification system followed by classification symbols) |
| G06N3/00, A61B5/117, G06F21/20, G06T7/00, H04L9/32 |

| Documentation searched other than minimum documentation to the extent that such documents are included in the fields searched |
|---|
| Jitsuyo Shinan Koho            1922-1996   Jitsuyo Shinan Toroku Koho    1996-2012 |
| Kokai Jitsuyo Shinan Koho      1971-2012   Toroku Jitsuyo Shinan Koho    1994-2012 |

| Electronic data base consulted during the international search (name of data base and, where practicable, search terms used) |
|---|
| |

### C. DOCUMENTS CONSIDERED TO BE RELEVANT

| Category* | Citation of document, with indication, where appropriate, of the relevant passages | Relevant to claim No. |
|---|---|---|
| A | JP 2006-338092 A  (NEC Corp.), 14 December 2006 (14.12.2006), paragraphs [0054] to [0080]; fig. 1 & US 2009/0087036 A1    & WO 2006/129551 A1 & CN 101189640 A | 1-11 |
| A | JP 2001-101406 A  (NTT Data Corp.), 13 April 2001 (13.04.2001), paragraphs [0048] to [0067]; fig. 6 (Family: none) | 1-11 |
| A | JP 2009-077221 A  (Toshiba Digital Media Engineering Corp.), 09 April 2009 (09.04.2009), paragraphs [0009] to [0021]; fig. 3 (Family: none) | 1-11 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11 April, 2012 (11.04.12) | 24 April, 2012 (24.04.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/054754

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2010-257060 A  (NTT Docomo Inc.), 11 November 2010 (11.11.2010), paragraphs [0022] to [0030]; fig. 2 (Family: none) | 1-11 |
| A | JP 2006-338263 A  (Nippon Telegraph and Telephone Corp.), 14 December 2006 (14.12.2006), paragraph [0017] (Family: none) | 1-11 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010128600 A